# EUROPEAN PATENT APPLICATION

(11) **EP 4 269 599 A1**
(43) Date of publication of application: **01.11.2023**
(21) Application number: 22833448.8
(22) Date of filing: 13.06.2022
(51) Int. Cl.: C12N 15/82, C07K 14/76, A61K 38/38, A61P 7/10, A23K 20/147

(54) **PLANT CELL LINE PRODUCING ALBUMIN AT HIGH YIELD AND USE THEREOF**

(30) Priority: 29.06.2021 KR 20210084727
(71) Applicant: Genecell Biotech Inc., Jeollabuk-do 55322 (KR)
(72) Inventor: KWON, Tae-Ho, Jeonju-si Jeollabuk-do 54905 (KR); YANG, Moon-Sik, Wanju-gun Jeollabuk-do 55360 (KR); KIM, Jin-A, Jeonju-si Jeollabuk-do 54956 (KR); KO, Suk-Min, Jeju-si Jeju-do 63126 (KR)
(74) Representative: Cabinet Nony
(86) International application number: PCT/KR2022/008308
(87) International publication number: WO 2023/277392

(57) **Abstract**

The present invention relates to a recombinant nucleic acid for producing albumin at high efficiency, an expression vector including the recombinant nucleic acid, and a rice suspension cell transformed with the expression vector. The rice suspension cell according to the present invention is useful for producing recombinant canine or feline albumin at high efficiency, and thus, by culturing the rice suspension cell, a large amount of recombinant canine or feline albumin can be safely produced at high efficiency. Moreover, it is expected that the veterinary composition for preventing, ameliorating, or treating hypoalbuminemia comprising the rice suspension cell of the present invention would greatly contribute to the resolving of the problems related to safety, social controversies, or the like that are caused by prescribing human serum albumin in veterinary care practice.

## Description

### Technical Field

The present invention relates to a plant cell line for producing albumin at high efficiency and a method for producing the plant cell line. More specifically, the present invention relates to rice suspension cell for producing at high efficiency recombinant albumin originating from dog or cat, a method for producing the rice suspension cell, and a veterinary composition for preventing, ameliorating, or treating hypoalbuminemia comprising the rice suspension cell or recombinant albumin protein derived from the rice suspension cell.

### Background Art

Human serum albumin (HSA) is the most abundant protein in human blood and mostly synthesized in liver. In human body, it plays an important physiological role. Specifically, it is known that human serum albumin not only delivers small molecules like fatty acid and low molecular weight proteins like bilirubin in blood and maintains the water balance between the inside and outside of blood vessel based on colloid osmosis pressure, but also is responsible for mot antioxidant functions in human body. Due to those various functional properties, in the medical field, human serum albumin is widely used as a pharmaceutical agent for treating hypovolemia, hypoproteinemia, shock, burns, massive bleeding, liver/kidney disorder, or the like.

Meanwhile, most human serum albumins used for medical practices are generally produced by separating them from blood taken from human body. Although it has been clinically used for a long period of time, human serum albumin has been recognized, among various types of blood agents, as a highly safe agent since there has been no case showing the propagation of infective substances like virus. However, DNA of Parvovirus B19, which is a DNA virus, was detected from human serum albumin agents, and, since even an albumin agent obtained after purification processes is still dependent on human plasma as a raw material, it is now gradually recognized that the unknown risks cannot be entirely excluded [Non Patent Document 001].

Aside from the issue relating to direct production using human blood, as a development of recombinant human serum albumin by utilizing various production systems like yeast and plant cells, studies have been made by many research groups including the applicant of the present invention, which eventually leads to the modern day commercialization of recombinant human serum albumin [Non Patent Document 002].

Meanwhile, in accordance with the rapid growth of pet care market in recent years, human serum albumin is also actually used in the animal clinic for treating hypoalbuminemia that is caused by various disorders like hepatic dysfunction, renal failure, burns, chronic inflammatory bowel disease, tumor, and lymphangiectasia. In general, human serum albumin is prescribed when blood albumin concentration is not more than 2.0 g/dL.

However, as it has been recently shown that the amino acid constitution of canine or feline serum albumin (CSA/FSA) has only 80% homology with human serum albumin, overcoming the 20% difference becomes an important issue for having safe prescription without causing any side effects.

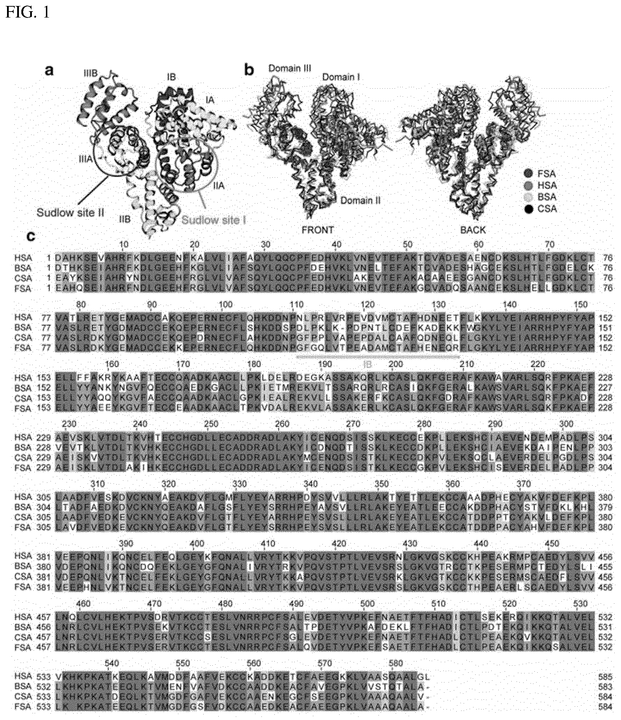

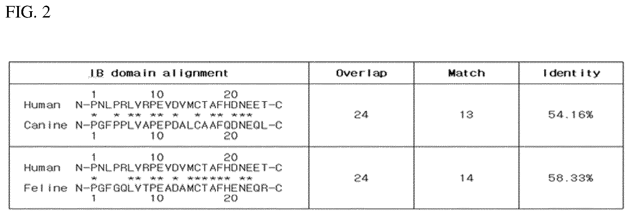

In mammals, non-conserved amino acid sequences are generally identified as an active antigen determinant (i.e., epitope) of an allergic reaction [Non Patent Document 003 and Non Patent Document 004]. It is known that IB domain (amino acid residues 110 to 133), which is a non-conservative sequence, is responsible for exhibiting the crucial difference in amino acid constitution between canine or feline serum albumin and human serum albumin, and it works as an active epitope of allergic antigenicity [Non Patent Document 005]. When the sequence itself is carefully compared between the IB domains, it is found that the main reason of having antibody production is based on the low homology of 54% for CSA and 58% for FSA, and thus it may be present as a critically negative factor for having the safe prescription (see, FIG. 1 and FIG. 2).

As an actual clinical case, there are reports showing that, when 25% human serum albumin is administered as a typical prescription to a dog or a cat which shows inflammatory bowel disease (IBD) or protein-losing enteropathy (PDE) as one of the major causes of hypoalbuminemia, serious acute side effects are caused so that the animal has to be killed or euthanized. In addition, according to the test in which human serum albumin is administered to a healthy dog or cat having no disease, severe edema, urticaria, skin lesion, and the like are shown [Non Patent Document 006]. Those side effects are recognized as an outcome of the immune response that is caused by the difference in amino acid sequence between human serum albumin and canine or feline serum albumin described in the above.

Taken together the results of prescribing human serum albumin which have been reported until now, it is concluded that, with a frequency of 30% or so, various and severe side effects like severe edema, inflammation, blood coagulation disorder, skin lesions caused by vasculitis, vomit, loss of appetite, weakness, high blood pressure, and death occur in dogs and cats which have been administered with human serum albumin [Non Patent Document 006 and Non Patent Document 007]. Moreover, the antibody produced as a result of administering a heterogeneous protein may also become a cause of nullifying the efficacy of albumin administered after secondary prescription [Non Patent Document 008].

Namely, due to the antibody produced against human serum albumin, an animal previously prescribed even once with human serum albumin may not enjoy the therapeutic effect of human serum albumin given in the secondary prescription and repeated administration, and also there is a high risk of having severe anaphylactic shock. Moreover, with regard to the prescription of human serum albumin in veterinary care practice, unlike US and European countries, regulations and legal bases relating to the use of human medicinal products for treating animal disease are not quite established in South Korea, and thus social controversies relating to this problem frequently arise in recent days.

However, since there is currently no animal exclusive albumin agent that can practically replace human serum albumin, human serum albumin prescriptions are still made in veterinary care practice while bearing the risk of side effects. In some veterinary clinics, a blood agent produced by using dog or cat blood is purchased and used. However, there are also several problems in terms of the social concerns and safety like problems associated with mass breeding of blood donor dog (i.e., dog for donating blood) and contaminated blood. On the contrary, in countries leading the way in animal welfare like US and European countries, only an officially approved plasma preparation which is produced from blood collected from donation by pet animals is allowed to be used in veterinary care practice.

Meanwhile, due to the increasing demand for blood preparation caused by rapid growth in pet care market in recent years, it becomes impossible to depend only on the blood preparation obtained by blood donation, and cost for producing canine serum albumin from blood plasma is higher than ever due to the purification processes, leading to a current increase in pet medical care cost.

### Prior Art Documents

### [Non Patent Document]

(Non Patent Document 001) Marano G, Vaglio S, Pupella S, et al. (2015) Human Parvovirus B19 and blood product safety: a tale of twenty years of improvements. Blood Transfus. 13:184-196.

(Non Patent Document 002) He Y, Ning T, Xie T, et al. (2011) Large-scale production of functional human serum albumin from transgenic rice seeds. Proc Natl Acad Sci USA108(47):19078-19083.

(Non Patent Document 003) Majorek KA, Porebski PJ, Dayal A, et al. (2012) Structural and immunologic characterization of bovine, horse, and rabbit serum albumins. Molecular Immunology 52(3-4):174-182.

(Non Patent Document 004) Tanabe S, Kobayashi Y, Takahata Y, Morimatsu F, Shibata R, Nishimura T (2002) Some human B and T cell epitopes of bovine serum albumin, the major beef allergen. Biochem Biophys Res Commun. 293(5):1348-1353.

(Non Patent Document 005) Pongprayoon P, Japrung D (2021) Revealing the structural dynamics of feline serum albumin. Structural Chemistry 32(1):69-77.

(Non Patent Document 006) Cohn LA, Kerl ME, Lenox CE, Livingston RS, Dodam JR. (2007) Response of healthy dogs to infusions of human serum albumin. Am J Vet Res. 68(6):657-663.

(Non Patent Document 007) Loyd KA, Cocayne CG, Cridland JM, Hause WR. (2016) Retrospective evaluation of the administration of 25% human albumin to dogs with protein-losing enteropathy: 21 cases (2003-2013). J Vet Emerg Crit Care (San Antonio). 26(4):587-592.

(Non Patent Document 008) Martin LG, Luther TY, Alperin DC, Gay JM, Hines SA. (2008) Serum antibodies against human albumin in critically ill and healthy dogs. J Am Vet Med Assoc. 232(7): 1004-1009.

### Detailed Description of the Invention

### Technical Problems to be Solved

The present invention is devised to meet the demand described in the above and solve the problems of conventional techniques, and object of the present invention is to provide a recombinant nucleic acid for producing albumin at high efficiency.

Another object of the present invention is to provide a rice suspension cell comprising the recombinant nucleic acid and a method of producing albumin at high efficiency by using the rice suspension cell.

Still another object of the present invention is to provide a veterinary composition for treating hypoalbuminemia comprising, as an effective component, the recombinant nucleic acid, rice suspension cell, or recombinant albumin protein originating from the recombinant nucleic acid and rice suspension cell.

Other object of the present application and technical subject to be achieved by the present invention are not limited by the objects described in the above, and they would be more evident from the following detailed description of the invention together with the claims and drawings that are included in the present specification. Furthermore, the technical subject to be achieved by the present invention but not described in the present specification may be clearly understood and figured out by a person who has common knowledge in the technical field to which the present invention pertains (hereinbelow, referred to as a "skilled person").

### Means for Solving Problems

To achieve the objects that are described in the above, the following inventions are provided.

The present invention provides a recombinant nucleic acid for producing albumin at high efficiency in which a nucleic acid sequence encoding signal peptide (SP) is attached to 5'-terminus of albumin gene.

According to one embodiment, the albumin gene in said recombinant nucleic acid may be a gene originating from a dog or a cat. More specifically, the albumin gene may be a gene including the nucleotide sequence of SEQ ID NO: 1 or SEQ ID NO: 2.

According to another embodiment, the signal peptide in said recombinant nucleic acid may be a peptide derived from rice amylase 3A (RAmy3A) or soybean vegetative storage protein (VspA). More specifically, the signal peptide may consist of the amino acid sequence of SEQ ID NO: 11 or SEQ ID NO: 12.

The present invention further provides an expression vector operably linked with a recombinant nucleic acid for producing albumin at high efficiency in which a nucleic acid sequence encoding signal peptide is attached to 5'-terminus of albumin gene.

According to one embodiment, the expression vector may include 5'-UTR, a nucleotide sequence encoding signal peptide, and a nucleotide sequence encoding mature peptide of canine albumin or feline albumin. More preferably, the expression vector may include any one nucleotide sequence selected from SEQ ID NO: 7 to SEQ ID NO: 10. The nucleotide sequence of SEQ ID NO: 7 to SEQ ID NO: 10 is a nucleotide sequence corresponding to 5'-UTR, a nucleotide sequence encoding signal peptide, and a nucleotide sequence encoding mature peptide of canine albumin or feline albumin. The nucleotide sequence of SEQ ID NO: 7 is a sequence in which 5'-UTR, a nucleotide sequence encoding rice amylase 3A (RAmy3A) signal peptide, and a nucleotide sequence encoding mature peptide of canine albumin are linked to one another in order, the nucleotide sequence of SEQ ID NO: 8 is a sequence in which 5'-UTR, a nucleotide sequence encoding soybean vegetative storage protein (VspA) signal peptide, and a nucleotide sequence encoding mature peptide of canine albumin are linked to one another in order, the nucleotide sequence of SEQ ID NO: 9 is a sequence in which 5'-UTR, a nucleotide sequence encoding rice amylase 3A (RAmy3A) signal peptide, and a nucleotide sequence encoding mature peptide of feline albumin are linked to one another in order, and the nucleotide sequence of SEQ ID NO: 10 is a sequence in which 5'-UTR, a nucleotide sequence encoding soybean vegetative storage protein (VspA) signal peptide, and a nucleotide sequence encoding mature peptide of feline albumin are linked to one another in order,

The present invention further provides a host cell comprising the expression vector described in the above.

As for the host cell allowing continuous expression of the expression vector of the present invention, any host cell well known in the pertinent art can be used. Examples of the host cell include a plant cell, *Escherichia coli,* yeast, and bacterial strain of *Bacillus* bacterial strain, but it is not limited thereto.

According to one embodiment of the present invention, the host cell may be a plant cell allowing the production of recombinant canine albumin or recombinant feline albumin at high efficiency. More specifically, the plant cell may be a rice suspension cell, but it is not limited thereto.

The present invention further provides a method for producing albumin at high efficiency including culturing a host cell comprising the expression vector. In this regard, the host cell may be a plant cell. More specifically, it may be a rice suspension cell, but it is not limited thereto.

The present invention further provides a veterinary composition for preventing or treating hypoalbuminemia comprising, as an effective component for producing albumin at high efficiency, a recombinant nucleic acid in which a nucleic acid sequence encoding signal peptide is attached to 5'-terminus of albumin gene; an expression vector operably linked with the recombinant nucleic acid; a host cell which produces, when transformed with the expression vector, recombinant canine albumin or recombinant feline albumin at high efficiency; or recombinant canine albumin or recombinant feline albumin derived from them.

According to one embodiment, the composition is used for administration to a dog or a cat, and it may be a composition which causes less side effects than the administration of human albumin. More specifically, the side effects may be edema, urticaria, or skin lesions.

The present invention still further provides an animal feed additive composition for preventing or ameliorating hypoalbuminemia comprising, as an effective component for producing albumin at high efficiency, a recombinant nucleic acid in which a nucleic acid sequence encoding signal peptide is attached to 5'-terminus of albumin gene; an expression vector operably linked with the recombinant nucleic acid; a host cell which produces, when transformed with the expression vector, recombinant canine albumin or recombinant feline albumin at high efficiency; or recombinant canine albumin or recombinant feline albumin derived from them.

### Advantageous Effects of the Invention

The recombinant nucleic acid, expression vector including the recombinant nucleic acid, and rice suspension cell comprising the vector, which are used for producing albumin at high efficiency in the present invention, may be used as an optimum means for safe and efficient production of a large amount of dog-exclusive recombinant CSA (rCSA; recombinant canine serum albumin) or cat-exclusive recombinant FSA (rFSA; recombinant feline serum albumin).

Moreover, the method of producing the recombinant canine or feline albumin by culturing the rice suspension cell of the present invention provides a technique which is unique in terms of the easiness of protein purification, safety, production cost, and the like.

Moreover, it is expected that the veterinary composition for preventing, ameliorating, or treating hypoalbuminemia comprising the rice suspension cell of the present invention or recombinant albumin protein derived from the suspension cell would greatly contribute to the resolving of the problems related to safety, social controversies, or the like that are caused by prescribing human serum albumin in veterinary care practice, which has been remained as a problem to be solved in the corresponding technical field.

### Brief Description of Drawings

FIG. 1 shows the result of comparing the protein structure and amino acid sequence of serum albumin among human, cow, dog, and cat.
FIG. 2 shows the result of comparing the amino acid constitution of IB domain, which is a non-conservative sequence found to be, as a result of the comparison of FIG. 1, responsible for the critical difference in amino acid sequence.
FIG. 3 shows the nucleotide sequence of a gene originating from canine albumin, which is used in the present invention.
FIG. 4 shows the nucleotide sequence of a gene originating from feline albumin, which is used in the present invention.
FIG. 5 shows the genetic map of a plant expression vector for expressing canine or feline albumin gene in monocot plant.
FIG. 6 shows the genetic map of a plant expression vector for expressing canine or feline albumin gene in dicot plant.
FIG. 7 shows the result of Western blot analysis in which the production level of canine albumin in rice callus, which has been introduced with canine albumin gene, is indicated.
FIG. 8 shows the result of Western blot analysis in which the production level of feline albumin in rice callus, which has been introduced with feline albumin gene, is indicated.
FIG. 9 shows the result of Western blot analysis in which the production level of canine albumin in tobacco plant, which has been introduced with canine albumin gene, is indicated.
FIG. 10 shows the result of Western blot analysis in which the production level of feline albumin in tobacco plant, which has been introduced with feline albumin gene, is indicated.
FIG. 11 shows the result of Western blot analysis in which the production level of canine albumin in potato plant, which has been introduced with canine albumin gene, is indicated.
FIG. 12 shows the result of Western blot analysis in which the production level of feline albumin in potato plant, which has been introduced with feline albumin gene, is indicated.
FIG. 13 shows the result of determining the production yield of canine albumin according to the present invention, in which the determination was made by ELISA analysis.
FIG. 14 shows the result of determining the production yield of feline albumin according to the present invention, in which the determination was made by ELISA analysis.
FIG. 15 shows the protein domain structure of canine albumin and feline albumin.
FIG. 16 shows the nucleotide sequence of KC11 gene in which 5'-UTR, a gene encoding rice amylase 3A (RAmy3A) signal peptide, and a gene encoding mature peptide of canine albumin are linked to one another.
FIG. 17 shows the nucleotide sequence of KC12 gene in which 5'-UTR, a gene encoding soybean vegetative storage protein (VspA), and a gene encoding mature peptide of canine albumin are linked to one another.
FIG. 18 shows the nucleotide sequence of KF11 gene in which 5'-UTR, a gene encoding rice amylase 3A (RAmy3A) signal peptide, and a gene encoding mature peptide of feline albumin are linked to one another.
FIG. 19 shows the nucleotide sequence of KF12 gene in which 5'-UTR, a gene encoding soybean vegetative storage protein (VspA), and a gene encoding mature peptide of feline albumin are linked to one another.
FIG. 20 shows the genetic map of pKC11, pKC12, pKF11, and pKF12, which are plant expression vectors for expressing the fused gene KC11, KC12, KF11, and KF12, respectively.
FIG. 21 shows the result of Western blot analysis in which the production level of recombinant canine albumin in rice suspension cell, which has been transformed with pKC11, is indicated.
FIG. 22 shows the result of Western blot analysis in which the production level of recombinant canine albumin in rice suspension cell, which has been transformed with pKC12, is indicated.
FIG. 23 shows the result of Western blot analysis in which the production level of feline albumin in rice suspension cell, which has been transformed with pKF11, is indicated.
FIG. 24 shows the result of Western blot analysis in which the production level of feline albumin in rice suspension cell, which has been transformed with pKF12, is indicated.
FIG. 25 shows (a) the result of determining the production yield of canine albumin in rice suspension cell KC11-5, KC11-16, KC11-20, and KC12-6, KC12-9, KC12-21 according to the present invention, in which the determination was made by ELISA analysis, and (b) the result of determining the production yield of feline albumin in rice suspension cell and rice suspension cell KF11-7, KF11-10, KF11-19, and KF12-8, KF12-15, KF12-24 according to the present invention, in which the determination was made by ELISA analysis.

### Best Mode(s) for Carrying out the Invention

To achieve the purpose of solving the several problems including safety issue, social controversies, or the like that are caused by prescribing human serum albumin in veterinary care practice, the present invention is completed on the basis of a recombinant nucleic acid for producing albumin at high efficiency, a vector including the recombinant nucleic acid, a rice suspension cell comprising it, and a veterinary composition for preventing, ameliorating, or treating hypoalbuminemia comprising it.

Moreover, the method of producing recombinant canine or feline albumin by culturing the rice suspension cell of the present invention provides a unique technique by which dog-exclusive recombinant CSA (rCSA; recombinant canine serum albumin) or cat-exclusive recombinant FSA (rFSA; recombinant feline serum albumin) can be safely and efficiently produced in a large amount.

Hereinbelow, the present invention is explained in greater detail in view of the examples. It is noted that the following examples include only part of the embodiments of the present invention and do not include all embodiments. It is evident that the context of the invention disclosed by the specification of the present invention is not limited to any specific example explained herein, but can be variously embodied by a skilled person in the technical field to which the present invention pertains. Thus, the disclosure of the invention described in the present specification is not limited to any specific example given herein, and it should be understood that modifications of the embodiments and other embodiments are also encompassed by the claims.

### Examples

### Example 1. Development of cell line for producing canine or feline albumin at high efficiency

### 1. Gene cloning and construction of expression vector

### (1) Canine or feline albumin gene and plasmid vector containing canine or feline albumin gene

To introduce the albumin gene to a plant expression vector based on genetic information of canine albumin (Uniprot P49822) and feline albumin (Uniprot P49064), 5'-terminus and 3'-terminus of canine albumin gene or feline albumin gene were introduced with Xbal site and KpnI site, respectively. Thermo Fisher Scientific was requested to carry out the synthesis of the gene.

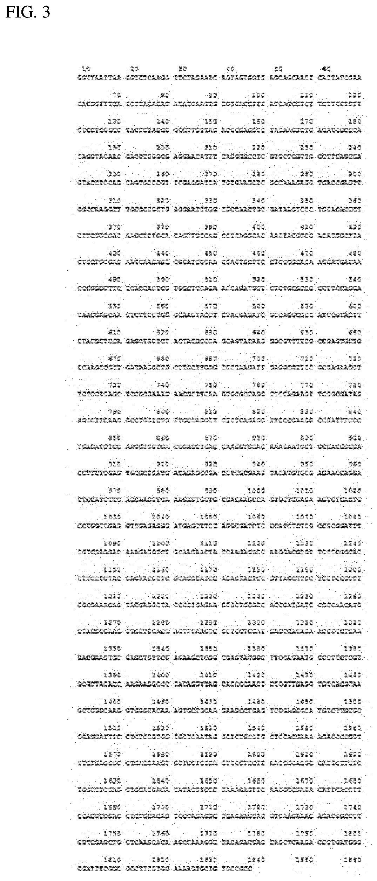

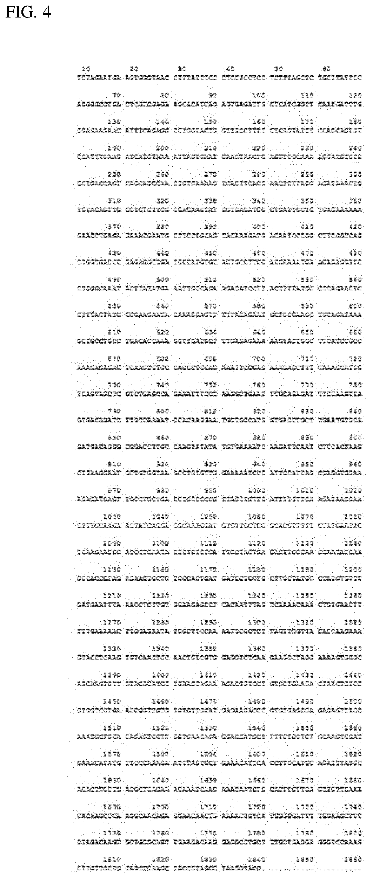

The nucleotide sequence of canine albumin gene (SEQ ID NO: 1) synthesized as described above was shown in FIG. 3, and the plasmid containing the canine albumin gene was named pKCT-1. The nucleotide sequence of feline albumin gene (SEQ ID NO: 2) synthesized in the present invention was shown in FIG. 4, and the plasmid containing the feline albumin gene was named pKFT-1.

### (2) Construction of plant expression vector for introducing canine or feline albumin gene to monocot plant

To induce the high expression of the canine albumin gene or feline albumin gene synthesized as described in the above in rice, which is a monocot plant, the gene was introduced to pMYN75, a vector developed by NBM Co., Ltd. for having high expression in monocot plant. pMYN75 plant expression vector is specifically expressed in rice suspension cells under glucose-depleted medium condition.

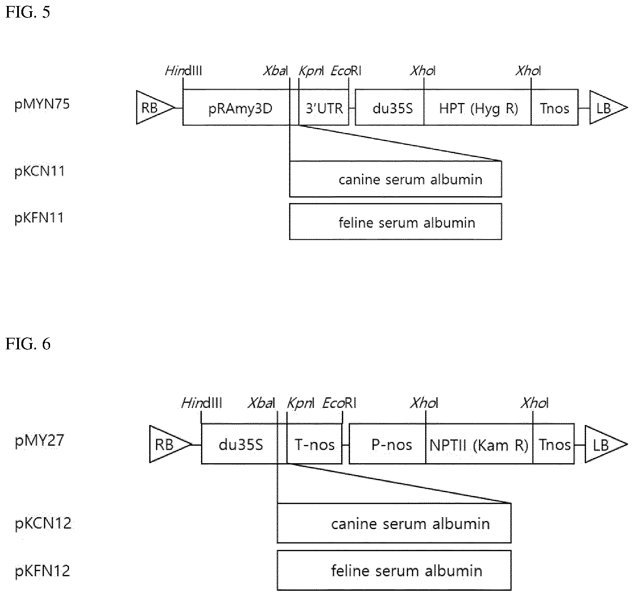

Plasmid pKCT-1 and plasmid pKFT-1, which contain the synthesized canine albumin gene and synthesized canine albumin gene, respectively, were first digested with KpnI and XbaI and then introduced separately to KpnI and XbaI sites of pMY75 vector so that canine albumin gene expression vector pKCN11 and feline albumin gene expression vector pKFN11 were produced. Detailed genetic map of expression vectors pKCN11 and pKFN11 is shown in FIG.5.

### (3) Construction of plant expression vector for introducing canine or feline albumin gene to dicot plant

To induce the high expression of the canine albumin gene or feline albumin gene synthesized as described in the above in tobacco or potato, which are a dicot plant, the gene was introduced to pMY27, a vector developed by NBM Co., Ltd. to have high expression in dicot plant.

Plasmid pKCT-1 and plasmid pKFT-1, which contain the synthesized canine albumin gene and synthesized canine albumin gene, respectively, were first digested with KpnI and XbaI and then introduced separately to KpnI and XbaI sites of pMY27 vector so that canine albumin gene expression vector pKCN12 and feline albumin gene expression vector pKFN12 were produced. Detailed genetic map of expression vectors pKCN12 and pKFN12 is shown in FIG.6.

### 2. Transformation and selection of plant cell line showing high productivity

In order to select an optimum plant and an optimum expression system for highly efficient production of albumin originating from dog or cat, the following experiments were carried out so as to establish a technique for producing canine or feline albumin in potato, tobacco, or rice. *(1) Agrobacterium* transformation of plant expression vector

Before carrying out *Agrobacterium-*mediated plant transformation, each of KCN11 and pKCN12; and pKFN11 and pKFN12, which have been prepared as described in the above, was transformed into *Agrobacterium tumefaciens* LBA4404 based on a triparental mating method.

*Escherichia coli* containing each of LBA4404, pKCN11, pKCN12, pKFN11 and pKFN12 plasmid, and *Escherichia coli* containing pRK2013, i.e., helper plasmid, which have been cultured under dark conditions for 2 days at 28°C, were cultured for 16 hours at 37°C. After that, each cell was collected in an amount of 1 ml, centrifuged, and washed 3 times with LB medium to remove the antibiotics included in the cells.

*Escherichia coli* containing each of pKCN11, pKCN12, pKFN11 and pKFN12 plasmid, and *Escherichia coli* containing LBA4404 and pRK2013 were collected in an amount of 100 µl for each, added to a fresh tube, and well absorbed in LB medium after thorough mixing. Then, the cells were cultured under dark conditions for 2 days at 28°C.

Two days after the culture, cells in LB medium were collected, diluted, and evenly absorbed on a solid medium containing rifampicin and kanamycin. Then, the cells were cultured under dark conditions for 3 days at 28°C. Three days after the culture, each grown colony was collected and examined to determine the gene incorporation. Specifically, by using a forward primer (SEQ ID NO: 3, F1: 5'-GCA AGT ACC TCT ACG AGA TC-3') and a reverse primer (SEQ ID NO: 4, R1: 5'-TGC GCT CGG TCT CAG GCT TC-3'), which are specific to canine albumin, and a forward primer (SEQ ID NO: 5, F2: 5'-CAG AGC GCA ACG AGT GCT TC-3') and a reverse primer (SEQ ID NO: 6, R2: 5'-TCT GTA CAG CAC TTC GTC AC-3'), which are specific to feline albumin, polymerase chain reaction (PCR) was carried out and then rice, tobacco, and potato were transformed with LBA4404 strain that has been confirmed by PCR to have the gene incorporation. Specific experimental procedures are explained in detail in the followings.

### (2) Rice transformation

To transform rice with canine albumin gene expression vector pKCN11 or feline albumin gene expression vector pKFN11, rice seeds (rice variety: Dongjin) with removed seed skin were subjected to surface sterilization using 70% ethanol, sterilized for 20 minutes in 2% Clorox, and sewn, about 16 seeds per medium, on N6CI solid medium (containing 30 g of sucrose, 4.4 g of N6 salt, 1 g of casamino acid, and 2 ppm 2,4-D, 0.8 % agar per liter), which is a callus induction medium. Three weeks later, only the milk-white calluses with diameter of 3 to 4 mm were separated, transferred to a fresh NCBI medium, and, three days later, used for transformation.

Rice callus was infected with LBA4404 strain containing pKCN11 or pKFN11, and then cultured in N6CO medium (containing 30 g of sucrose, 4.4 g of N6 salt, 1 g of casamino acid, and 10 g glucose and 2 ppm 2,4-D per liter), which contains 100 µM acetosyringone, under dark conditions for 3 days at 25°C.

After that, the rice callus was collected, washed with water first, and additionally washed with water containing 500 µg/ml cefotaxime. By adding the callus on a filter paper, extra LBA4404 strains were then removed. The cells were added on N6S medium (N6CI medium added with 50 µg/ml hygromycin B and 500 µg/ml cefotaxime) and cultured for 3 to 4 weeks. Only the newly growing callus while showing resistance to the antibiotic hygromycin was separated, transferred to a fresh medium, and then allowed to proliferate.

### (3) Tobacco transformation

Tobacco transformation was carried out by using the leaf-disc method, which has been previously reported by Horsch et. al. First, leaves of tobacco (variety name: BY-2) were sterilized with 70% ethanol and then sodium hypochlorite, for 1 minute and 5 minutes, respectively, washed with sterile water, cut to a size of 1 cm, and then inoculated with LBA4404 strain containing pKCN12 or pKFN12. The cells were added to MS-NB medium (Murashige-Skoog, 1 mg/L 6-BAP, 0.1 mg/L NAA) and co-cultured.

Three days later, the cells were transferred to MS-NB medium containing the antibiotic carbenicillin to remove the extra *Agrobacterium* and induce a callus simultaneously. Ten days later, the cells were added to MS-NB medium containing canamycin (100 mg/L) and carbenicillin (500 mg/L) to proceed with the selection of transformants. Adventitious buds formed from the callus were transferred to the above MS medium containing antibiotics, and then a plant with roots formed by regeneration was selected. The plants were cultivated in a growth chamber under 12-hour light conditions at 25°C. Following the acclimation period, the plants were transplanted in a pot and then cultivated.

### (4) Potato transformation

Potato transformation was carried out by using the tuber-disc method, which has been previously reported by Stiekema et. al. Tuber skin of a potato (variety name: Soomi) was removed first and subjected to surface sterilization with sodium hypochlorite (chlorine concentration: 1%), and then washed 4 times with sterile water. The resulting tuber was cut by a sterile cork borer (diameter: 1 cm) and further cut to have a thickness of 3 mm to prepare a tuber-disc. The tuber-disc was impregnated, for 15 minutes, in LBA4404-suspended MS liquid medium containing pKCN12 or pKFN12 for inoculation, and then added to the MS solid medium and co-cultured under 16-hour light conditions for 3 days at 21°C.

After that, the tuber-disc was washed with MS liquid medium containing canamycin (50 mg/L) and carbenicillin (500 mg/L) and then co-cultured under 16-hour light conditions at 21°C in MS solid medium containing the above antibiotics and plant growth regulator (0.1 mg/L IAA, 0.1 mg/L GA3, 0.1 mg/L ABA, and 2 mg/L zeatin riboside) to proceed with removal of extra *Agrobacterium* and selection of transformants. The tuber-disc was transferred to a fresh medium with an interval of 2 weeks, and, from the plant having newly-formed shoots, the shoots were cut off to promote root formation and the plant was transferred to MS solid medium (added with 50 mg/L canamycin) which has not been added with any plant growth regulator. The transformant was then allowed to grow and, following the acclimation period, it was transplanted in a pot and cultivated.

### 3. Selection of plant cell line having high productivity of albumin

### (1) Selection of rice cell line having high productivity of albumin

Albumin production from a transformed rice callus which has been introduced with expression vector pKCN11 containing canine albumin gene or a transformed rice callus which has been introduced with expression vector pKFN11 containing feline albumin gene was determined by Western blot analysis using an antibody specific to canine or feline albumin.

Specifically, 10 g of the transformed rice callus which has been introduced with canine or feline albumin gene were admixed with 10 ml of extraction buffer (50 mM Tris-Cl pH 8.2, 0.5% Triton X-100, 30 mM NaCl, 1 mM PMSF), and then ground using a pestle and mortar to extract proteins. Western blot analysis was carried out by using iBlot^{™} 2 Gel Transfer Device of Fisher Scientific according to the manufacturer's experimental procedure. As a primary antibody against canine albumin, dog-specific antibody by Bethyl Laboratories (i.e., Bethyl A40-113A) was used. As a primary antibody against feline albumin, cat-specific antibody by Bethyl Laboratories (i.e., Bethyl A20-109A) was used.

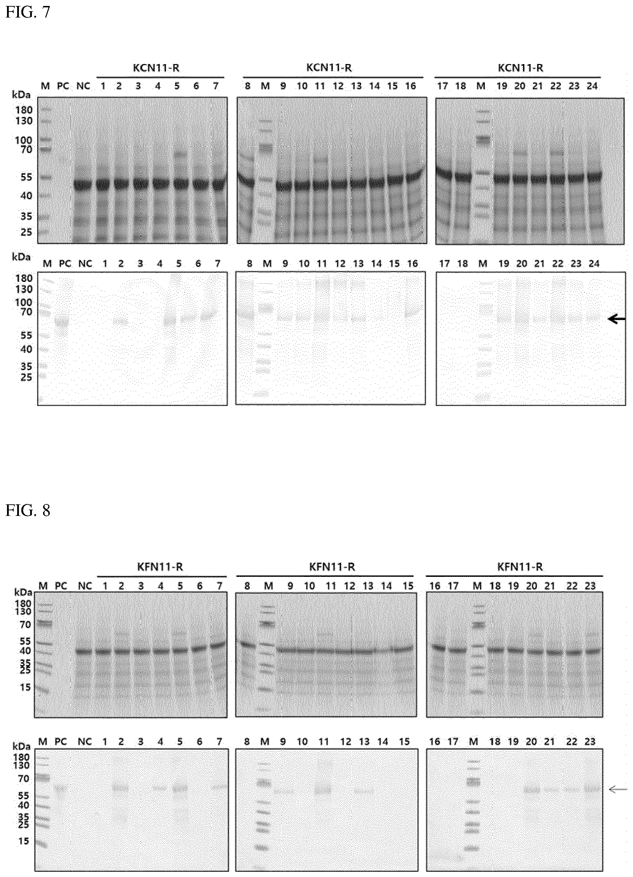

As a result of the Western blot analysis, a strong band believed to be canine albumin was found from samples KCN11-R5 and KCN11-R22 among the samples of the rice callus transformed with canine albumin gene. Accordingly, KCN11-R5 and KCN11-R22 calluses were selected as a rice elite line having high productivity of canine albumin (FIG. 7). Moreover, a strong band believed to be feline albumin was found from samples KFN11-R5, KFN11-R11 and KFN11-R23 among the samples of the rice callus transformed with feline albumin gene. Accordingly, KFN11-R5, KFN11-R11 and KFN11-R23 calluses were selected as a rice elite line having high productivity of feline albumin (FIG. 8).

### (2) Selection of tobacco cell line having high productivity of albumin

Albumin production from a transformed tobacco plant which has been introduced with expression vector pKCN12 containing canine albumin gene or a transformed tobacco plant which has been introduced with expression vector pKFN12 containing feline albumin gene was determined by Western blot analysis using an antibody specific to canine albumin (Bethyl A40-113A) or an antibody specific to feline albumin (Bethyl A20-109A). Specifically, 10 g of the leaves of each transformed plant were used as a sample, and the experiment was carried out in the same manner as the above "Selection of rice cell line having high productivity of albumin."

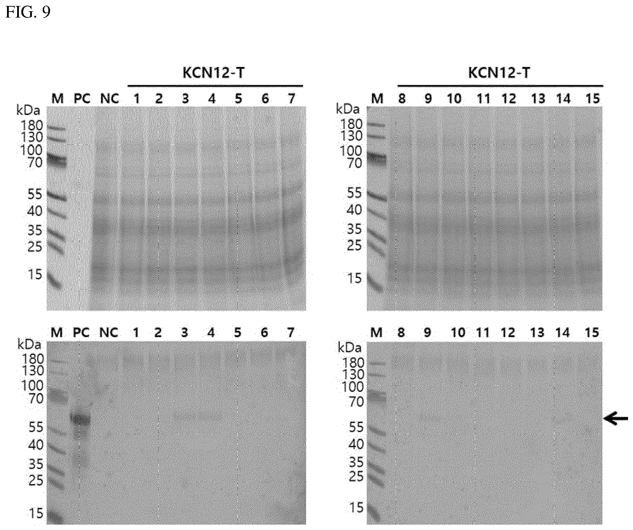

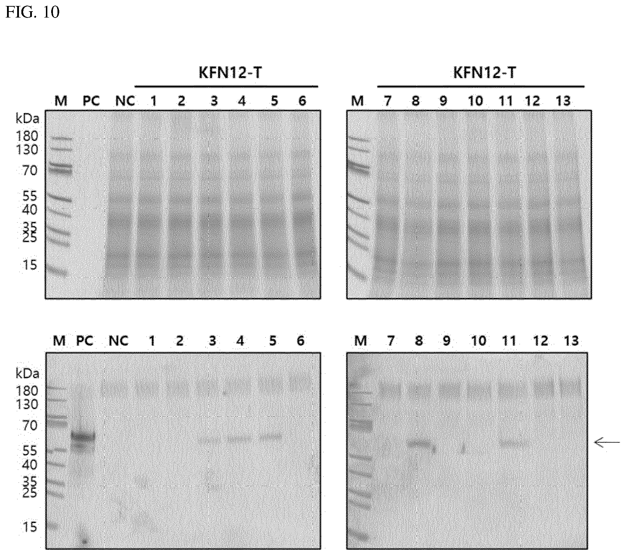

As a result of carrying out the Western blot analysis, a strong band believed to be canine albumin was found from samples KCN12-T3 and KCN12-T4 among the samples of the tobacco plant transformed with canine albumin gene. Accordingly, KCN12-T3 and KCN12-T4 plants were selected as a tobacco elite line having high productivity of canine albumin (FIG. 9). Moreover, a strong band believed to be feline albumin was found from samples KFN12-T8 and KFN12-T11 among the samples of the tobacco plant transformed with feline albumin gene. Accordingly, KFN12-T8 and KFN12-T11 plants were selected as a tobacco elite line having high productivity of feline albumin (FIG. 10).

### (3) Selection of potato cell line having high productivity of albumin

Albumin production from a transformed potato plant which has been introduced with expression vector pKCN12 containing canine albumin gene or a transformed potato plant which has been introduced with expression vector pKFN12 containing feline albumin gene was determined by Western blot analysis using an antibody specific to canine albumin (Bethyl A40-113A) or an antibody specific to feline albumin (Bethyl A20-109A). Specifically, 10 g of the leaves of each transformed plant were used as a sample, and the experiment was carried out in the same manner as the above "Selection of rice cell line having high productivity of albumin."

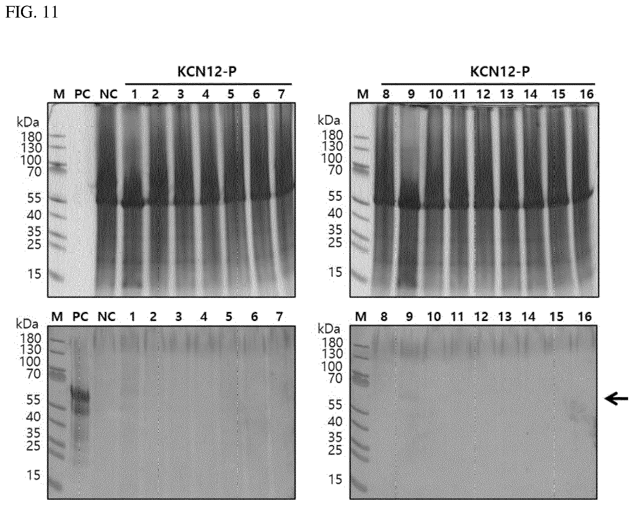

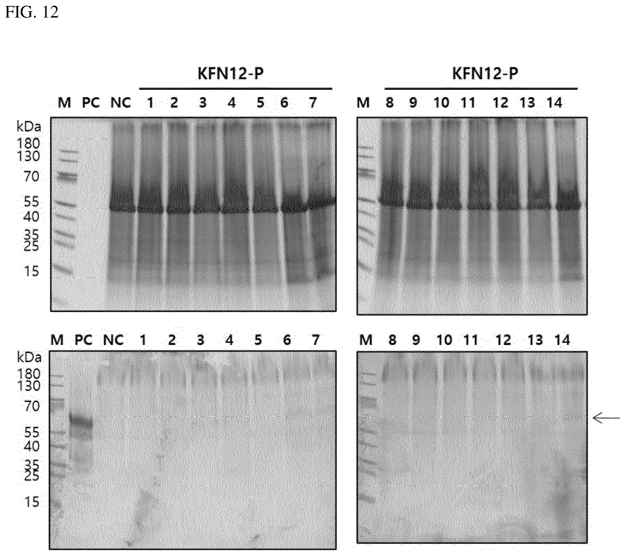

As a result of carrying out the Western blot analysis, a strong band believed to be canine albumin was found from samples KCN12-P1 and KCN12-P9 among the samples of the potato plant transformed with canine albumin gene. Accordingly, KCN12-P1 and KCN12-P9 plants were selected as a potato elite line having high productivity of canine albumin (FIG. 11). Moreover, a strong band believed to be feline albumin was found from samples KFN12-P6 and KFN12-P14 among the samples of the potato plant transformed with feline albumin gene. Accordingly, KFN12-P6 and KFN12-P14 plants were selected as a potato elite line having high productivity of feline albumin (FIG. 12).

### Example 2. Determination of production yield of cell line having high productivity of canine or feline albumin

Albumin production yield of the plant cell lines having high productivity of canine or feline albumin, which have been selected in Example 1, was determined by using ELISA method, and specific explanation are given below.

### 1. Induction of suspension cell from plant callus or plant having high productivity of canine or feline albumin

### (1) Induction of suspension cell from transformed rice callus having high productivity of canine or feline albumin

KCN11-R5 and KCN11-R22 cell lines (FIG. 7), which have been selected in Example 1 as a rice callus having high productivity of canine albumin, and KFN11-R5, KFN11-R11 and KFN11-R23 cell lines (FIG. 8) , which have been selected in Example 1 as a rice callus having high productivity of feline albumin, were inoculated to N6CI liquid medium (containing 30 g of sucrose, 4.4 g of N6 salt, 1 g of casamino acid and 2 ppm 2,4-D per liter) and subcultured with an interval of 3 weeks under dark conditions at 25°C to induce suspension cells. 1/5 of the culture was transferred every 7 days to a fresh medium, and subjected to subculture.

### (2) Induction of suspension cell from transformed tobacco plant having high productivity of canine or feline albumin

KCN12-T3 and KCN12-T4 (FIG. 9) leaves, which have been selected in Example 1 as a tobacco transformant having high productivity of canine albumin, and KFN12-T8 and KFN12-T11 (FIG. 10) leaves, which have been selected in Example 1 as a tobacco transformant having high productivity of feline albumin, were sterilized for 1 minute with 70% ethanol and for 5 minutes in sodium hypochlorite, washed with sterile water, cut to a size of 1 cm, and then added to MS solid medium (containing 2 mg/L 2,4-dichlorophenoxyacetic acid, 0.02 mg/L kinetin, 3% sucrose and 0.8% agar) and subcultured with an interval of 3 weeks under 3,000 lux conditions at 25°C to induce calluses. Six to eight weeks later, the induced calluses were inoculated to 50 ml MS liquid medium from which agar has been removed, and cultured in a rotary incubator at 25°C, 110 rpm. 1/5 of the suspension cells was transferred every 7 days to a fresh medium, and subjected to subculture.

### (3) Induction of suspension cell from transformed potato plant having high productivity of canine or feline albumin

KCN12-P1 and KCN12-P9 (FIG. 11) leaves, which have been selected in Example 1 as a potato transformant having high productivity of canine albumin, and KFN12-P6 and KFN12-P14 (FIG. 12) leaves, which have been selected in Example 1 as a potato transformant having high productivity of feline albumin, were sterilized for 1 minute with 70% ethanol and for 5 minutes in sodium hypochlorite, washed with sterile water, cut to a size of 1 cm, and then added to MS solid medium (containing 3 mg/L NAA, 2 mg/L IBA, 3% sucrose and 0.8% agar) and subcultured with an interval of 3 weeks under dark conditions at 25°C to induce calluses. Six to eight weeks later, the induced calluses were inoculated to 50 ml MS liquid medium from which agar has been removed, and cultured in a rotary incubator at 25°C, 110 rpm. 1/5 of the suspension cells was transferred every 7 days to a fresh medium, and subjected to subculture.

### 2. Determination of albumin production yield using ELISA

### (1) Sample preparation for carrying out ELISA

Each of rice suspension cell KCN11-R5 and KCN11-R22; tobacco suspension cell KCN12-T3 and KCN12-T4; and potato suspension cell KCN12-P1 and KCN12-P9, which have high productivity of canine albumin, and rice suspension cell KFN11-R5, KFN11-R11 and KFN11-R23; tobacco suspension cell KFN12-T8 and KFN12-T11; and potato suspension cell KFN12-P6 and KFN12-P14, which have high productivity of feline albumin, all maintained by liquid culture, was added in a suitable amount on a filter paper of a sterile vacuum filter funnel. By applying vacuum, liquid medium was removed, and each suspension cell after removal of the liquid medium was collected in an amount of 10 g. The tobacco suspension cell and potato suspension cell were then cultured by adding them to 50 ml of a liquid medium which has been used for culture of the suspension cell. The rice suspension cell was cultured by adding it to 50 ml of a liquid medium used for culture of the suspension cell while sucrose is removed from the liquid medium components. Each of those suspension cells was repeatedly cultured three times. The liquid medium on day 5 after the culture was then collected and quantification of albumin content was carried out.

For ELISA analysis, 1 ml culture of each suspension cell was centrifuged for 10 minutes at 4°C and 200xg conditions to separate the culture supernatant from pellet. Dialysis was carried out overnight at 4°C against PBS, and the dialyzed culture supernatant was employed as a sample. For determination of the production amount of canine albumin, canine albumin ELISA kit (ab277078, Abcam, UK) was used. For determination of the production amount of feline albumin, feline albumin ELISA Kit (DEIABL383, Creative Diagnostics, USA) was used to carry out the quantification analysis. The analytical process was repeatedly carried out 3 times according to the protocol included in each kit.

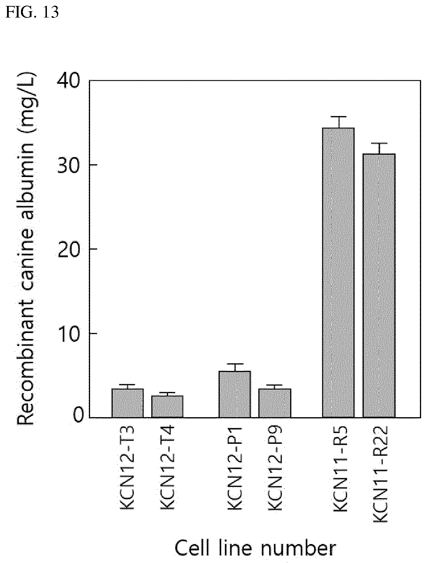

Results of the ELISA analysis for rice suspension cell (KCN11-R5, KCN11-R22), tobacco suspension cell (KCN12-T3, KCN12-T4), and potato suspension cell (KCN12-P1, KCN12-P9), which have high productivity of canine albumin, are shown in FIG. 13. Albumin production yield of the tobacco suspension cell having high productivity of canine albumin was about 3 mg/L at most and albumin production yield of the potato suspension cell having high productivity of canine albumin was about 5 mg/L at most. On the other hand, canine albumin production yield of the rice suspension cell was as high as 34 mg/L, which is about 10 times higher than the tobacco suspension cell and about 7 times higher than the potato suspension cell, indicating very high production yield.

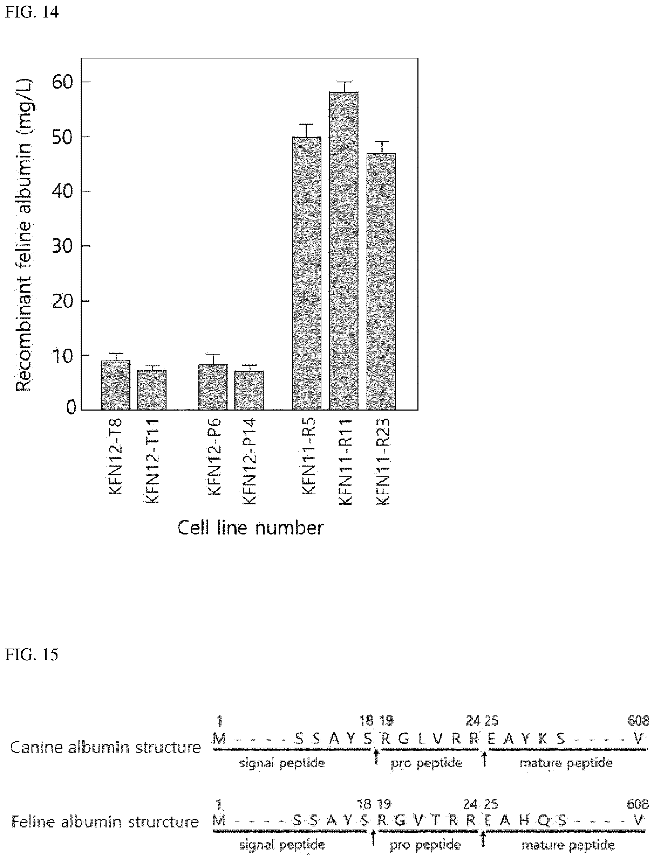

Results of the ELISA analysis for rice suspension cell (KFN11-R5, KFN11-R11, KFN11-R23), tobacco suspension cell (KFN12-T8, KFN12-T11), potato suspension cell (KFN12-P6, KFN12-P14), which have high productivity of feline albumin, are shown in FIG. 14. Albumin production yield of the tobacco suspension cell having high productivity of feline albumin was about 9 mg/L at most and albumin production yield of the potato suspension cell having high productivity of feline albumin was about 9 mg/L at most. On the other hand, feline albumin production yield of the rice suspension cell was as high as 58 mg/L, which is about 6.5 times higher than the tobacco suspension cell and potato suspension cell, indicating very high production yield.

It was found based on Example 2.2 above that, for producing recombinant canine or feline albumin in a plant, introducing a plant expression vector including canine or feline albumin gene to rice callus is most preferable.

### 3. Determination of N-terminus amino acid sequence of canine albumin or feline albumin derived from rice

N-terminus amino acid sequence of canine albumin or feline albumin, which has been produced by the aforementioned rice suspension cell, was determined.

Rice suspension cell KCN11-R5 culture, which produces canine albumin, and rice suspension cell KFN11-R11 culture, which produces feline albumin, were taken, each in an amount of 100 ml, and filtered through a filter with pore size of 0.45 µm. After subjecting the filtrate to molecular weight cut-off using a 30 kDa hollow fiber filter, the concentrate was applied to HiScreen Blue Sepharose FF (Cytiva, 28978243) column to separate and purify albumin according to the protocol provided by the manufacturer. Life Science Laboratory (South Korea) was requested to carry out the amino acid sequencing of the purified canine albumin and feline albumin by using Edman degradation method.

Based on the information of canine albumin (Uniprot P49822) and feline albumin (Uniprot P49064) that are used in Example 1 of the present invention, it was recognized that both the canine albumin and feline albumin consist of a signal peptide (1 to 18), a propeptide (19 to 24), and a mature peptide (25 to 608).

As a result of the amino acid sequencing, two types of the amino acid sequence, i.e., R-G-L-V-R and L-V-R-R-E, are found as the N-terminus amino acid sequence of the canine albumin produced in rice suspension cell. It was also found that R-G-L-V-R includes the whole propeptide as shown in the diagram of FIG. 15 while L-V-R-R-E includes only the 21^{st} to the 24^{th} amino acids (FIG. 15). Moreover, as a result of the amino acid sequencing, two types of the amino acid sequence, i.e., R-G-V-T-R and V-T-R-R-E, are found as the N-terminus amino acid sequence of the feline albumin produced in rice suspension cell. It was also found that R-G-V-T-R includes the whole propeptide as shown in FIG. 15 while V-T-R-R-E includes only the 21^{st} to the 24^{th} amino acids.

The results described above indicate that the canine albumin or feline albumin, which is produced by the rice suspension cell of the present invention, is expressed as an immature protein in which the propeptide sequence is not completely removed. Accordingly, to have the same protein expression as the mature protein in natural state present in blood of a dog or a cat, further experiments were carried out.

### Example 3. Method for producing in rice suspension cell the same recombinant canine albumin or feline albumin as mature protein in natural state

As a result of the determination of Example 2, it was found that the N-terminus of the canine albumin and feline albumin produced in rice suspension cell is different from that of the mature protein of natural type originating from a dog or a cat. As such, an attempt was made to establish a technique for mass production of recombinant albumin having the same N-terminus sequence as the natural type.

Signal peptide (SP) is a peptide sequence which consists of 3 to 60 amino acids present in protein molecule. It plays a role of guiding the transport and localization of a protein synthesized in cytoplasm. Specific sequences for guiding the transport and localization to a cell organelle like nucleus, Golgi body, mitochondria, and chlorophyll are known. Among them, endoplasmic reticulum SP for extracellular secretion (i.e., transport to endoplasmic reticulum) is a sequence which contains 5 to 10 hydrophobic amino acids that are present at N-terminus of a protein molecule. The endoplasmic reticulum SP is utilized for having a system of efficient production (secretion) of various foreign proteins, in which selection of an optimum SP and corresponding accuracy (i.e., accurate cleavage) are the highly critical factors for enhancing the production efficiency of a target protein.

The production efficiency of a target protein varies greatly depending on the type of SP introduced to an expression vector. Because there is no sequence conserved across biospecies, it is necessary to conduct, based on combination of various SPs, a search for an optimum sequence which exhibits the highest production efficiency.

### 1. Selection of optimum signal peptide

### (1) Selection of optimum signal peptide for producing recombinant canine or feline albumin

It is known that, during the process of forming a tertiary structure, the propeptide of mammalian albumin is cleaved by furine as an endopeptidase to yield a mature albumin protein. As it has been determined in Example 2 of the present invention, when recombinant canine or feline albumin is produced by using rice suspension cell, the propeptide cleavage is not achieved since furine as an endopeptidase present in mammals does not exist in rice. As a result, the N-terminus sequence of the produced albumin is different from that of the natural type mature protein of a dog or a cat.

Accordingly, a search was made to obtain an optimum signal peptide which enables the expression of the natural type mature canine or feline protein in the rice suspension cell of the present invention. Specifically, prior art techniques used for producing a recombinant protein in plant were investigated and, by using the database related to protein signal peptide sequence (http://proline.bic.nus.edu.s g/spdb), 13 signal peptide sequences were extracted. The extracted 13 types of the signal peptide sequence were linked to the mature peptide of canine or feline albumin, and then, by using SignalP-5.0 server (http://www.cbs.dtu.dk/services/SignalP/), the property of accurate cleavage of the signal peptide sequence was examined.

Results of determining the probability of having cleavage of the signal peptide sequence by using SignalP-5.0 program are given in Table 1 and Table 2, respectively. As a result of linking the 13 types of signal peptide amino acid sequence to the mature canine or feline peptide sequence and determining the probability of having signal peptide cleavage, it was found that the highest probability of having signal peptide cleavage is obtained, for both canine albumin and feline albumin, when linking is made with rice amylase 3A (RAmy3A) signal peptide sequence (MGKQMAALCGFLLVALLWLTPDVAHA, SEQ ID NO: 11) or soybean vegetative storage protein (VspA) signal peptide sequence (MKMVVLVFFVATILVAWQCHA, SEQ ID NO: 12).

**[Table 1]**

| Determination of cleavage site of canine albumin depending on signal peptide sequence | | | |
|---|---|---|---|
| Signal peptide | Source | Signal peptide sequence (SEQ ID NO:) | Cleavage site (probability score) |
| RAmy3A | α-Amylase 3A (*Oryza sativa)* | MGKQMAALCGFLLVAL LWLTPDVAHA (11) | AHA^{∨}EAYK (0.8929) |
| CRT | Calreticulin (*Nicotiana tabacum*) | MATQRRANPSSLHLITVF SLLVAVVSA (13) | VSA^{∨}EAYK (0.3162) |
| PGLA | polygalacturonase (*Malus domestica)* | MALKTQLLWSFVVVFVV SFSTTSCSG (14) | CSG^{v}EAYK (0.3593) |
| BAmyA | α-Amylase type A (*Hordeum vulgare*) | MGKNGSLCCFSLLLLLLL AGLASG (15) | ASG^{v}EAYK (0.6323) |
| BAmyB | α-Amylase type B (*Hordeum vulgare*) | MANKHLSLSLFLVLLGLS ASLASG (16) | ASG^{∨}AYK (0.5221) |
| RAamy3D | α-Amylase 3D (*Oryza sativa*) | MKNTSSLCLLLLVVLCSL TCNSGQA (17) | GQA^{v}EAYK (0.2921) |
| Gt13a | Glutenin (*Oryza sativa*) | MASINRPIVFFTVCLFLLC NGSLA (18) | SLA^{∨}EAYK (0.4125) |
| EXT | Extensin domain (*N. benthamiana*) | MGKMASLFATLLVVLVS LSLASESSA (19) | SSA^{∨}EAYK (0.3121) |
| Pr1 | Pathogenesis related protein 1 (*N. benthamiana*) | MLPFSLLVSTLLLFLVISH SCRA (20) | CRA^{∨}EAYK (0.6785) |
| VspA | Storage protein (*Glycine max*) | MKMVVLVFFVATILVA WQCHA (12) | CHA^{∨}EAYK (0.7217) |
| PDI | Protein disulphide isomerase (*Medicago truncatula*) | MAKNVSIFGLLFSLLALV PSQIFA (21) | IFA^{∨}EAYK (0.5940) |
| OsCIN 1 | Hypothetical protein (*Oryza sativa*) | MGTRLLALAPWLLLLLL QLAGA (22) | AGA^{∨}EAYK (0.3514) |
| Os33KD | Hypothetical protein (*Oryza sativa*) | MAALSQLVLVTAFLAAA LLPLGMAA (23) | MAA^{∨}EAYK (0.0000) |
| Remarks) Amino acid sequence EAYK indicates mature peptide sequence of canine | | | |
| albumin | | | |

**[Table 2]**

| Determination of cleavage site of feline albumin depending on signal peptide sequence | | | |
|---|---|---|---|
| Signal peptide | Source | Amino acid sequence | Cleavage site (probability score) |
| RAmy3A | α-Amylase 3A (*Oryza sativa*) | MGKQMAALCGFLLVA LLWLTPDVAHA | AHA^{∨}EAHQ (0.9027) |
| CRT | Calreticulin (*Nicotiana tabacum*) | MATQRRANPSSLHLITV FSLLVAVVSA | VSA^{∨}EAHQ (0.3261) |
| PGLA | polygalacturonase (*Malus domestica*) | MALKTQLLWSFVVVFV VSFSTTSCSG | CSG^{∨}EAHQ (0.3690) |
| BAmyA | α-Amylase type A (*Hordeum vulgare*) | MGKNGSLCCFSLLLLLL LAGLASG | ASG^{∨}EAHQ (0.6146) |
| BAmyB | α-Amylase type B (*Hordeum vulgare*) | MANKHLSLSLFLVLLGL SASLASG | ASG^{∨}EAHQ (0.5104) |
| RAamy3D | α-Amylase 3D (*Oryza sativa*) | MKNTSSLCLLLLVVLCS LTCNSGQA | GQA^{∨}EAHQ (0.2974) |
| Gt13a | Glutenin (*Oryza sativa*) | MASINRPIVFFTVCLFLL CNGSLA | SLA^{∨}EAHQ (0.4920) |
| EXT | Extensin domain (*N. benthamiana*) | MGKMASLFATLLVVLV SLSLASESSA | SSA^{∨}EAHQ (0.4054) |
| Pr1 | Pathogenesis related protein 1 (*N. benthamiana*) | MLPFSLLVSTLLLFLVIS HSCRA | CRA^{∨}EAHQ (0.6934) |
| VspA | Storage protein (*Glycine max*) | MKMVVLVFFVATILVA WQCHA | CHA^{∨}EAHQ (0.7356) |
| PDI | Protein disulphide isomerase (*Medicago truncatula*) | MAKNVSIFGLLFSLLAL VPSQIFA | IFA^{∨}EAHQ (0.6168) |
| OsCIN 1 | Hypothetical protein (*Oryza sativa*) | MGTRLLALAPWLLLLL LQLAGA | AGA^{∨}EAHQ (0.3321) |
| Os33KD | Hypothetical protein (*Oryza sativa*) | MAALSQLVLVTAFLAA ALLPLGMAA | MAA^{∨}EAHQ (0.0000) |
| Remarks) Amino acid sequence EAHQ indicates mature peptide sequence of feline albumin | | | |

### 2. Gene synthesis and preparation of expression vector

### (1) Gene synthesis and preparation of expression vector

As it has been determined in above Example 2 of the present invention, it was found that, when albumin is produced in rice suspension cell, cleavage is not made to have the same N-terminus as the albumin present in blood of a dog or a cat, and part of the propeptide of canine or feline albumin (i.e., 4 to 6 amino acids) is present at the N-terminus of the mature peptide of canine or feline albumin.

In view of this result, expression was made after directly linking rice amylase 3A (RAmy3A) signal peptide or soybean vegetative storage protein (VspA) signal peptide, which have been selected in Example 3 of the present invention, to the N-terminus of the mature peptide of canine or feline albumin such that the cleavage can occur to yield the same N-terminus as the albumin present in blood of a dog or a cat.

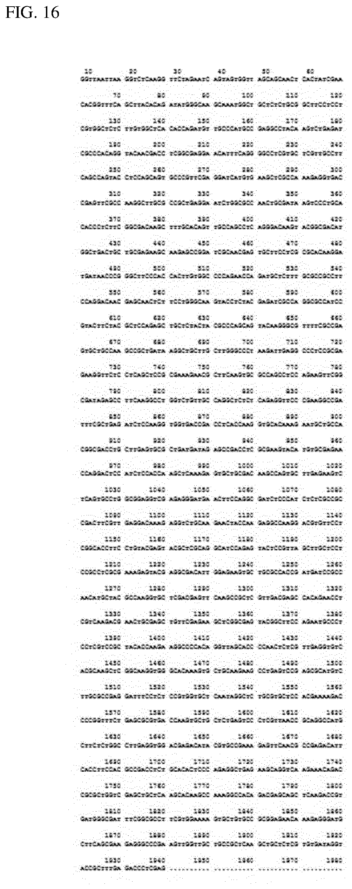

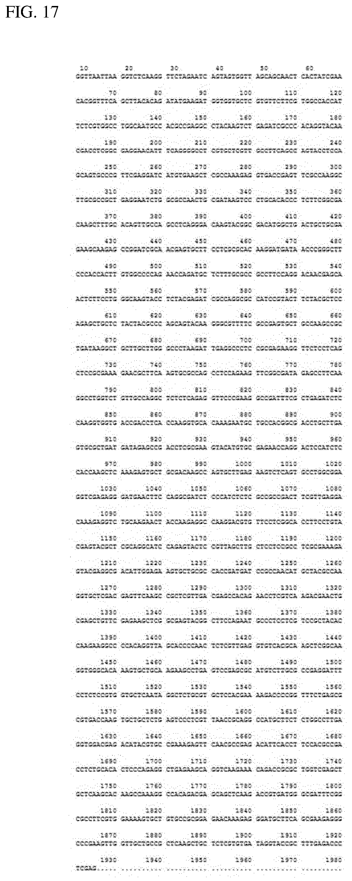

As for the canine albumin gene and feline albumin gene, optimization to rice codon was made by using GeneArt^{™} GeneOptimizer^{™} program provided in the webpage of Thermo Fisher Scientific, and then Thermo Fisher Scientific was requested to synthesize the genes. Nucleotide sequence of the gene (KC11), in which 5'-UTR, rice amylase 3A (RAmy3A) signal peptide, and a gene encoding the mature peptide of canine albumin are fused to one another, is represented in FIG. 16 (SEQ ID NO: 7). Plasmid including the thus-synthesized recombinant canine albumin gene was named "pKCT-3". Nucleotide sequence of the gene (KC12), in which 5'-UTR, soybean vegetative storage protein A (VspA) signal peptide, and a gene encoding the mature peptide of canine albumin are fused to one another, is represented in FIG. 17 (SEQ ID NO: 8). Plasmid including the thus-synthesized recombinant canine albumin gene was named "pKCT-4".

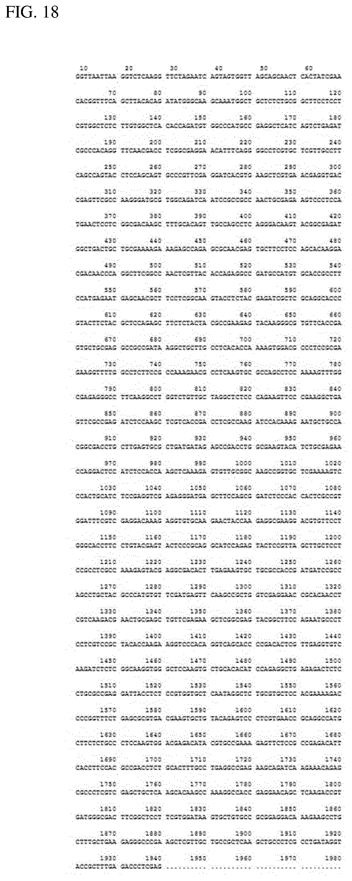

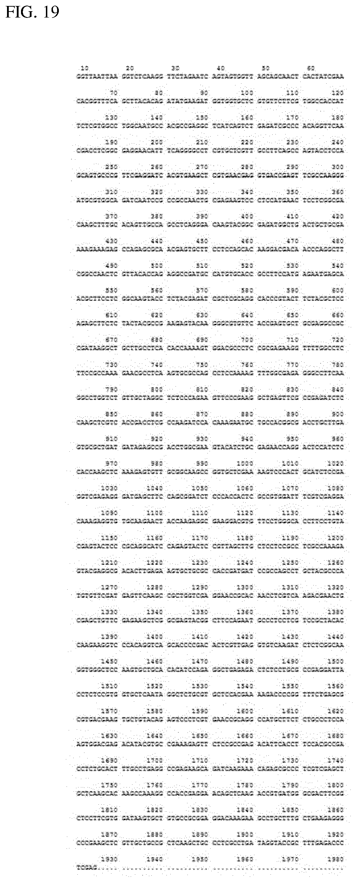

Similarly, nucleotide sequence of the gene (KF11), in which 5'-UTR, RAmy3A signal peptide, and a gene encoding the mature peptide of feline albumin are fused to one another, is represented in FIG. 18 (SEQ ID NO: 9). Plasmid including the thus-synthesized recombinant feline albumin gene was named "pKFT-3". Nucleotide sequence of the gene (KF12), in which 5'-UTR, VspA signal peptide, and a gene encoding the mature peptide of feline albumin are fused to one another, is represented in FIG. 19 (SEQ ID NO: 10). Plasmid including the thus-synthesized recombinant feline albumin gene was named "pKFT-4".

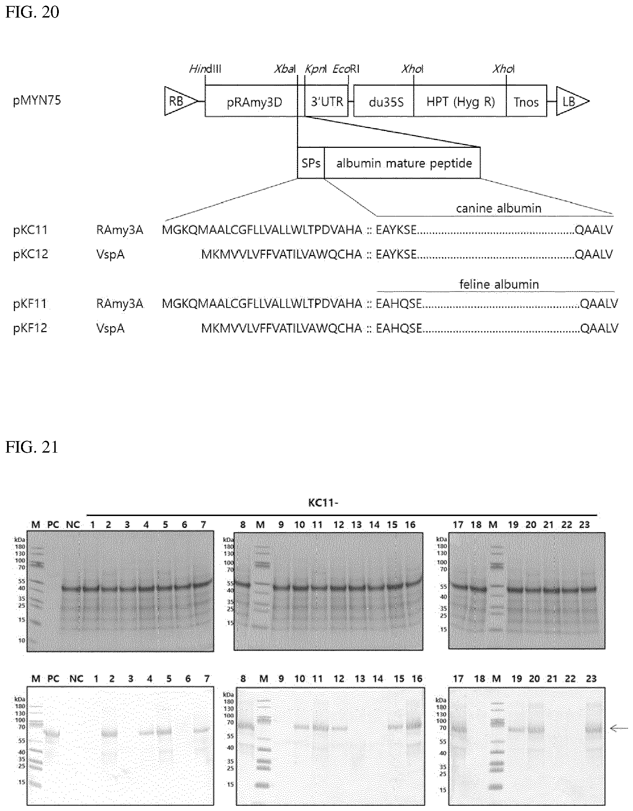

To induce the high expression of each of the recombinant canine albumin and recombinant feline albumin gene, which have been synthesized above, in rice as a monocot plant, each gene was introduced to pMYN75, which is a vector for high expression in monocot plant used in Example 1 of the present invention. After cleaving pKCT-3 and pKCT-4, which include the recombinant canine albumin gene optimized to plant codon, by using KpnI and XbaI, respectively, they were introduced to the KpnI and XbaI site, respectively, of pMYN75 vector to produce recombinant canine albumin gene expression vector pKC11 and pKC12. Detailed genetic map of those vectors is given in FIG. 20.

Furthermore, pKFT-3 and pKFT-4, which include the recombinant feline albumin gene optimized to plant codon, were cleaved by using KpnI and XbaI, respectively, and then they were introduced to the KpnI and XbaI site, respectively, of pMYN75 vector to produce feline albumin gene expression vector pKF11 and pKF12. Detailed genetic map of those vectors is given in FIG. 20.

### 3. Rice transformation and selection of cell line having high productivity

### (1) Rice transformation and selection of cell line having high productivity

Each of pKC11, pKC12, pKF11, and pKF12 vector described in the above was introduced to rice along with *Agrobacterium* by following the method described in Example 1. Productivity of albumin in the transformed rice callus was then carried out by Western blot analysis described in Example 1.

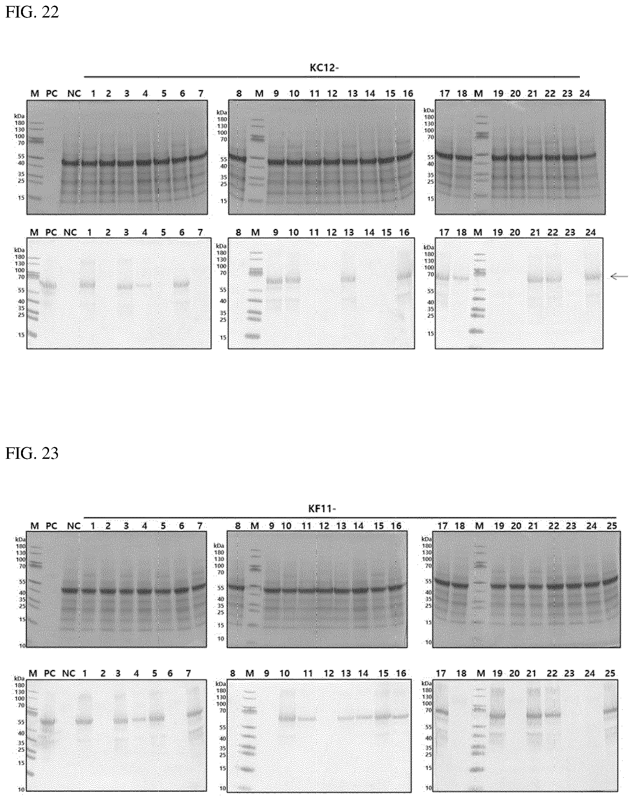

As a result of examining the 23 lines of callus which have been selected after transformation with pKC11 including a gene in which RAmy3A signal peptide and the mature peptide of canine albumin are fused to each other, strong band was found from the cell lines of KC11-5, KC11-16 and KC11-20, and thus they were selected as a cell line having high productivity of recombinant canine albumin (FIG. 21). Furthermore, as a result of examining the 23 lines of callus which have been selected after transformation with pKC12 including a gene in which VspA signal peptide and the mature peptide of canine albumin are fused to each other, strong band was found from the cell lines KC12-6, KC12-9 and KC12-21, and thus they were selected as a cell line having high productivity of recombinant canine albumin (FIG. 22).

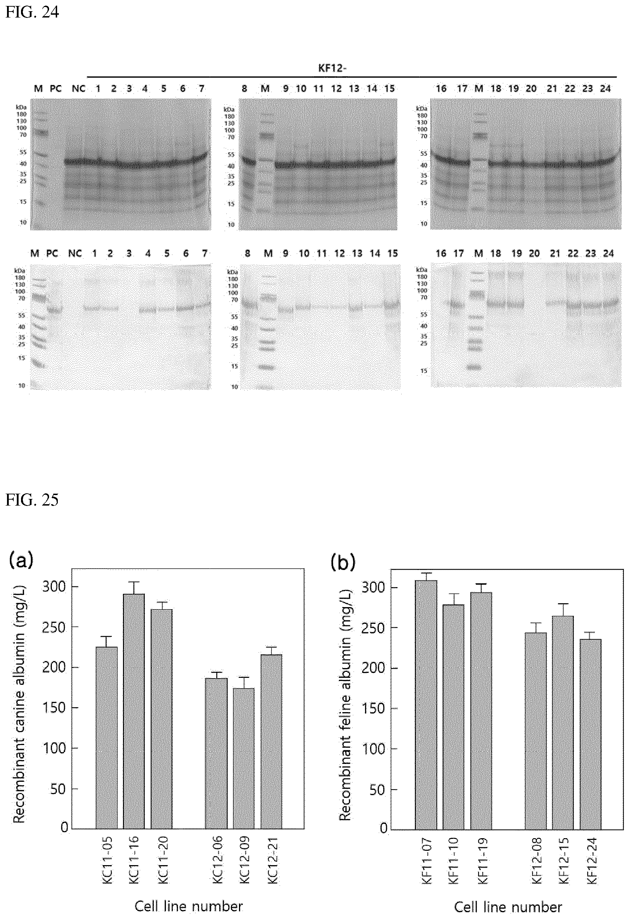

Moreover, as a result of examining the 25 lines of callus which have been selected after transformation with pKF11 including a gene in which RAmy3A signal peptide and the mature peptide of feline albumin are fused to each other, strong band was found from the cell lines of KF11-7, KF11-10 and KF11-19, and thus they were selected as a cell line having high productivity of recombinant feline albumin (FIG. 23). Furthermore, as a result of examining the 24 lines of callus which have been selected after transformation with pKF12 including a gene in which VspA signal peptide and the mature peptide of feline albumin are fused to each other, strong band was found from the cell lines KF12-8, KF12-15 and KF12-24, and thus they were selected as a cell line having high productivity of recombinant feline albumin (FIG. 24).

### 4. Determination of albumin production yield

### (1) Determination of production yield of recombinant canine albumin by using ELISA

According to the same ELISA method as the method described in above Example 2 of the present invention, production yield of recombinant canine albumin by rice suspension cell KC11-5, KC11-16, KC11-20, KC12-6, KC12-9 and KC12-21 having high productivity of recombinant canine albumin, which have been selected in the above, was determined, and the results are given in FIG. 25(a).

As a result of determining the production yield of recombinant canine albumin by the transformed suspension cell line introduced with pKC11, KC11-5 suspension cell line exhibits the production yield of about 250 mg/L, KC11-16 suspension cell line exhibits the production yield of about 290 mg/L, and KC11-20 suspension cell line exhibits the production yield of about 270 mg/L. Furthermore, as a result of determining the production yield of recombinant canine albumin by the transformed suspension cell line introduced with pKC12, KC12-6 suspension cell line exhibits the production yield of about 180 mg/L, KC12-9 suspension cell line exhibits the production yield of about 170 mg/L, and KC12-21 suspension cell line exhibits the production yield of about 220 mg/L. With regard to the production yield of recombinant canine albumin depending on type of a signal peptide, it was found that the production yield is about 50 mg/L higher on average when rice amylase 3A (RAmy3A) signal peptide is used.

When the above results are compared to the production yield of about 34 mg/L determined in Example 2 of the present invention, which has been obtained from KCN11-R5 suspension cell line having high productivity of recombinant canine albumin in which a signal peptide of canine albumin is used, it is found that the production yield is improved by about 8 to 10 times by using RAmy3A signal peptide, and also by about 5 to 6.5 times by using VspA signal peptide.

The inventors of the present invention deposited KC11-16 rice cell line, which has high productivity of recombinant canine albumin as completed in Example 3, with Korean Collection for Type Cultures (KCTC) (Accession No. KCTC 14618BP). The inventors also found based on Example 3 that, to have mass production of recombinant canine albumin in plant, use of rice amylase 3A (RAmy3A) signal peptide or soybean vegetative storage protein A (VspA) signal peptide is more effective than use of a canine signal peptide, and preferably, use of RAmy3A signal peptide is more effective.

### (2) Determination of production yield of recombinant feline albumin by using ELISA

According to the same ELISA method as the method described in above Example 2 of the present invention, production yield of recombinant feline albumin by rice suspension cell KF11-7, KF11-10, KF11-19, KF12-8, KF12-15 and KF12-24 having high productivity of recombinant feline albumin, which have been selected in the above, was determined, and the results are given in FIG. 25(b).

As a result of determining the production yield of recombinant feline albumin by the transformed suspension cell line introduced with pKF11, KF11-7 suspension cell line exhibits the production yield of about 310 mg/L, KF11-10 suspension cell line exhibits the production yield of about 280 mg/L, and KF11-19 suspension cell line exhibits the production yield of about 290 mg/L, showing production yield of about 290 mg/L on average. Furthermore, as a result of determining the production yield of recombinant feline albumin by the transformed suspension cell line introduced with pKF12, KF12-8 suspension cell line exhibits the production yield of about 240 mg/L, KF12-15 suspension cell line exhibits the production yield of about 260 mg/L, and KF12-24 suspension cell line exhibits the production yield of about 230 mg/L, showing production yield of about 243 mg/L. With regard to the production yield of recombinant feline albumin depending on type of a signal peptide, it was found that the production yield is about 47 mg/L higher on average when rice amylase 3A (RAmy3A) signal peptide is used.

When the above results are compared to the production yield of about 58 mg/L determined in Example 2 of the present invention, which has been obtained from KFN11-R11 suspension cell line having high productivity of recombinant feline albumin in which a signal peptide of feline albumin is used, it is found that the production yield is improved by about 5 times by using RAmy3A signal peptide, and also by about 4 times by using VspA signal peptide.

The inventors of the present invention deposited KF11-7 cell line, which has high productivity of recombinant feline albumin as completed in Example 3, with Korean Collection for Type Cultures (KCTC) (Accession No. KCTC 114617BP). The inventors also found based on Example 3 that, to have mass production of recombinant feline albumin in plant, use of rice amylase 3A (RAmy3A) signal peptide or soybean vegetative storage protein A (VspA) signal peptide is more effective than use of a feline signal peptide, and preferably, use of RAmy3A signal peptide is more effective.

### 5. Determination of N-terminus amino acid sequence of recombinant albumin

N-terminus amino acid sequence of recombinant canine albumin which is produced by KC11-16 introduced with pKC11 and having high productivity of recombinant canine albumin, or by KC12-21 cell line introduced with pKC12 and having high productivity of recombinant canine albumin was determined by the same method as the method described in Example 2. As a result, it was found that all recombinant canine albumin produced by cell lines KC11-16 and KC12-21 have the N-terminus amino acid sequence of E-A-Y-K-S, and the result indicates the same N-terminus sequence as that of mature canine albumin protein present in natural state in dog blood.

Furthermore, N-terminus amino acid sequence of recombinant feline albumin which is produced by KF11-07 introduced with pKF11 and having high productivity of recombinant feline albumin, or by KF12-15 cell line introduced with pKF12 and having high productivity of recombinant feline albumin was determined by the same method as the method described in Example 2. As a result, it was found that all recombinant feline albumin produced by cell lines KF11-07 and KF12-15 have the N-terminus amino acid sequence of E-A-H-Q-S, and the result indicates the same N-terminus sequence as that of mature feline albumin protein present in natural state in cat blood.

### Example 4. Efficacy evaluation of recombinant canine or feline albumin

Efficacy of the recombinant canine or feline albumin produced in Example 3 above was evaluated by an animal test. Huvet Co., Ltd. was requested to carry out the animal test.

For treating a sick animal suffering from hypoalbuminemia in vet clinic, an albumin preparation manufactured for use for human is presently used, and side effects like face edema or the like are reported. The recombinant canine albumin used in the present invention is derived from a plant and prepared to be actually applied to a sick animal in vet clinic, and, in the present test, dogs and cats which are actually admitted as sick animal in vet clinic were employed as a subject.

### (1) Efficacy of recombinant canine albumin in hypoalbuminemia model

Male beagle dog, which is 10-month old and has body weight of 10 to 10.6 kg, was obtained from Orient Bio. Veterinary checkup with regard to general health state of all animals was carried out first and the animal was acclimated and kept for 7 days. The animal room was constantly maintained at temperature of 24±2°C, humidity of 40 to 60%, and light/dark cycle of 12 hours. As for the animal food, the animal was allowed to have free access to a commercially available food exclusive for dogs and also purified water. The test was carried out after obtaining the approval by Institutional Animal Care and Use Committee (IACUC) of Research Laboratory of Huvet Co., Ltd. (Approval No. HV 2022-002). The test group consists of Control (n=3) in which physiological saline (0.9% normal saline) is administered after bleeding 8% of whole blood, and albumin administration group (n=3) in which recombinant canine albumin is administered after bleeding 8% of whole blood.

Test animal obtained after acclimation was anesthetized by injecting a mixture of ketamine (ketamine hydrochloride, 50 mg/ml, YUHAN) and rompun injection solution (xylazine hydrochloride, 23.32 mg/ml, Bayer Korea), each in an amount of 0.1 ml/kg and 0.1 ml/kg, respectively, and, after securing a catheter in cephalic vein, bleeding was slowly induced by using a 10 cc syringe. Bleeding was allowed to occur till to have 70 ml of blood, which is 8% of the whole blood. Physiological saline (50 ml) and recombinant canine albumin (2 g/10 ml/vial) were admixed in an amount of 2 g/5 kg to meet the body weight requirement of test animal, and intravenously injected, at rate of 70 ml/h by using an infusion pump, to a subject confirmed to have low amount of albumin after bleeding. Blood was taken before bleeding, after bleeding, and Day 1, Day 3, and Day 7 after administering the recombinant canine albumin, and a change in complete blood count (BC-2800Vet, Mindray) and serum biochemistry profile (PT10V, Samsung) was analyzed.

**[Table 3]**

| Change in major blood counts | | | | | | |
|---|---|---|---|---|---|---|
| Test group | Test item | Before bleeding (Baseline) | After bleeding | Day 1 | Day 3 | Day 7 |
| Recombinant canine albumin | Hematocrit (%) | 40.9±2.3 | 33.3±2.8 | 38.2±3.3 | 40.0±4.2 | 41.6±3.5 |
| | Globulin (g/dl) | 3.5±0.2 | 3.3±0.1 | 3.5±0.2 | 3.4±0.3 | 3.6±0.3 |
| | Albumin (g/dl) | 2.7±0.3 | 2.3±0.2 | 2.6±0.1 | 2.8±0.2 | 3.0±0.1 |
| | ALB/GLOB | 0.77 | 0.71 | 0.76 | 0.83 | 0.86 |
| | Total protein (g/dl) | 7.1±0.1 | 6.5±0.2 | 7.1±0.4 | 7.4±0.4 | 7.5±0.3 |
| Control | Hematocrit (%) | 53.1±4.2 | 54.6±2.8 | 49.9±3.5 | 49.9±3.6 | 50.4±2.7 |
| | Globulin (g/dl) | 2.7±0.2 | 2.9±0.3 | 3.0±0.1 | 3.0±0.1 | 2.9±0.2 |
| | Albumin (g/dl) | 3.2±0.3 | 2.9±0.2 | 3.0±0.3 | 3.0±0.3 | 3.0±0.2 |
| | ALB/GLOB | 1.18 | 1.00 | 1.00 | 1.00 | 0.93 |
| | Total protein (g/dl) | 5.9±0.2 | 5.6±0.3 | 6.3±0.3 | 6.0±0.2 | 5.9±0.4 |
| 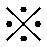 Normal range | | | | | | |
| Hematocrit (%): 39 to 56, Globulin (g/dl): 2.3 to 4.6, | | | | | | |
| Albumin (g/dl): 2.2 to 3.9, Total protein (g/dl): 5.3 to 8.4 | | | | | | |

In the above Table 3, the result of analyzing a change in blood count before bleeding (i.e., baseline), after bleeding, and Day 1, Day 3, and Day 7 after administering albumin is shown. The following Tables 4 and 5 show the result obtained by conversion into percentage (%) based on the count before bleeding.

**[Table 4]**

| Change in blood count of Control (saline) (based on the count of before bleeding, %) | | | | | |
|---|---|---|---|---|---|
| Category (%) | Control | | | | |
| | Before bleeding (Baseline) | Before administration (after bleeding) | After administration (Dav 1) | After administration (Dav 3) | After administration (Dav 7) |
| Hematocrit | 100 | 85.8 (▼ 14.2) | 94.0 (▲ 8.2) | 94.0 (=) | 94.9 (▲ 0.9) |
| Globulin | 100 | 107.1 (▲ 7.1) | 111.1 (▲ 4.0) | 111.1 (=) | 107.4 (▼ 3.7) |
| Albumin | 100 | 90.6 (▼ 9.4) | 93.8 (▲ 3.2) | 93.8 (=) | 93.8 (=) |
| Total Protein | 100 | 94.9 (▼ 5.1) | 106.7 (▲ 11.8) | 101.7 (▼ 5.0) | 100.0 (▼ 1.7) |
| ▲ and ▼ indicate an increase or a decrease of the percentage value of the corresponding column compared to the percentage value of immediately preceding column in each line. | | | | | |

**[Table 5]**

| Change in blood count of albumin administration group (based on the count of before bleeding, %) | | | | | |
|---|---|---|---|---|---|
| Category (%) | Recombinant canine albumin administration group | | | | |
| | Before bleeding (Baseline) | Before administration (after bleeding) | After administration (Day 1) | After administration (Day 3) | After administration (Day 7) |
| Hematocrit | 100 | 81.4 (▼ 18.6) | 93.6 (▲ 12.2) | 98.0 (▲ 4.4) | 101.7 (▲ 3.7) |
| Globulin | 100 | 94.2 (▼ 5.8) | 100.0 (▲ 5.8) | 98.1 (▼ 1.9) | 102.8 (▲ 4.7) |
| Albumin | 100 | 86.4 (▼ 13.6) | 98.8 (▲ 12.4) | 103.7 (▲ 4.9) | 113.6 (▲ 2.4) |
| Total Protein | 100 | 91.5 (▼ 8.5) | 100.5 (▲ 9) | 104.3 (▲ 3.7) | 105.7 (▲ 2.0) |
| ▲ and ▼ indicate an increase or a decrease of the percentage value of the corresponding column compared to the percentage value of immediately preceding column in each line. | | | | | |

It was found that the large difference between Control and the recombinant canine albumin administration group is shown on Day 1 after injecting the test material, and, at that time, the increase rate of blood albumin is about 9.2% higher in the albumin administration group compared to Control, and it is maintained up to Day 7. Moreover, other than the above blood counts, abnormal changes like higher inflammation count were not observed from the blood count test after injecting the test material. According to the serum biochemistry profile, functional numbers of liver and kidney were found to be normal. Moreover, according to the urine test carried out by collecting urine by abdominal paracentesis, no abnormalities were observed, either. Side effects like face edema due to albumin injection were also not observed. Based on the above results, it is considered that the administration of recombinant canine albumin helps to have a higher albumin value without any side effect.

### (2) Efficacy of recombinant feline albumin in hypoalbuminemia model

Male cat, which is 10-month old and has body weight of 4.5 to 5 kg, was obtained from Orient Bio. Veterinary checkup with regard to general health state of all animals was carried out first and the animal was acclimated and kept for 7 days. The animal room was constantly maintained at temperature of 24±2°C, humidity of 40 to 60%, and light/dark cycle of 12 hours. As for the animal food, the animal was allowed to have free access to a commercially available food exclusive for cats and also purified water. The test was carried out after obtaining the approval by Institutional Animal Care and Use Committee (IACUC) of Research Laboratory of Huvet Co., Ltd. (Approval No. HV 2022-002). The test group consists of Control (n=3) in which physiological saline (0.9% normal saline) is administered after bleeding 8% of whole blood and albumin administration group (n=3) in which recombinant feline albumin is administered after bleeding 8% of whole blood.

Test animal obtained after acclimation was anesthetized by injecting a mixture of ketamine (50 mg/ml) and rompun injection solution (23.32 mg/ml), each in an amount of 0.1 ml/kg and 0.1 ml/kg, respectively, and, after securing a catheter in cephalic vein, bleeding was slowly induced by using a 10 cc syringe. Bleeding was allowed to occur till to have 35 ml of blood, which is 8% of the whole blood. Physiological saline (25 ml) and recombinant feline albumin (2 g/10 ml/vial) were admixed in an amount of 2 g/5 kg to meet the body weight requirement of test animal, and intravenously injected, at rate of 35 ml/h by using an infusion pump, to a subject confirmed to have low amount of albumin after bleeding. Blood was taken before bleeding, after bleeding, and Day 1, Day 3, and Day 7 after administering the recombinant feline albumin, and a change in complete blood count and serum biochemistry profile was analyzed.

**[Table 6]**

| Change in major blood counts | | | | | | |
|---|---|---|---|---|---|---|
| Test group | Test item | Before bleeding (Baseline) | After bleeding | Day 1 | Day 3 | Day 7 |
| Recombinant feline albumin | Hematocrit (%) | 40.9±1.4 | 33.3±1.4 | 38.2±4.8 | 40.1±3.9 | 41.6±2.8 |
| | Globulin (g/dl) | 3.8±0.2 | 3.6±0.1 | 3.9±0.3 | 4.0±0.3 | 4.0±0.3 |
| | Albumin (g/dl) | 2.8±0.2 | 2.4±0.2 | 2.8±0.1 | 3.0±0.2 | 3.0±0.2 |
| | ALB/GLOB | 0.73 | 0.68 | 0.73 | 0.75 | 0.76 |
| | Total protein (g/dl) | 7.1±0.2 | 6.5±0.2 | 7.1±0.3 | 7.4±0.2 | 7.5±0.1 |
| Control | Hematocrit (%) | 50.2±2.3 | 41.3±3.3 | 46.8±4.5 | 49.1±3.4 | 50.3±2.8 |
| | Globulin (g/dl) | 4.5±0.2 | 4.1±0.3 | 4.1±0.2 | 4.3±0.3 | 4.5±0.1 |
| | Albumin (g/dl) | 3.0±0.1 | 2.7±0.3 | 2.8±0.2 | 3.0±0.1 | 2.9±0.2 |
| | ALB/GLOB | 0.7 | 0.7 | 0.7 | 0.7 | 0.6 |
| | Total protein (g/dl) | 7.5±0.2 | 6.4±0.2 | 6.9±0.3 | 7.3±0.2 | 7.7±0.2 |
| Normal range | | | | | | |
| 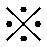 Hematocrit (HCT, %): 28 to 49, Globulin (GLOB, g/dl): 2.6 to 5.1, | | | | | | |
| Albumin (ALB, g/dl): 2.2 to 4.1, Total protein (TP, g/dl): 5.8 to 9.1 | | | | | | |

In the above Table 6, the result of analyzing a change in blood count before bleeding (i.e., baseline), after bleeding, and Day 1, Day 3, and Day 7 after administering albumin is shown. The following Tables 7 and 8 show the result obtained by conversion into percentage (%) based on the count before bleeding'

**[Table 7]**

| Change in blood count of Control (saline) (based on the count of before bleeding, %) | | | | | |
|---|---|---|---|---|---|
| Category (%) | Control | | | | |
| | Before bleeding (Baseline) | Before administration (after bleeding) | After administration (Day 1) | After administration (Day 3) | After administration (Day 7) |
| Hematocrit | 100 | 82.3 (▼ 17.7) | 93.2 (▲ 10.9) | 97.8 (▲ 4.6) | 100.2 (▲ 2.4) |
| Globulin | 100 | 91.1 (▼ 8.9) | 91.1 (=) | 95.6 (▲ 4.4) | 100 (▲ 4.4) |
| Albumin | 100 | 90 (▼ 10) | 93.3 (▲ 3.3) | 100 (▲ 6.7) | 96.7 (▼ 3.3) |
| Total protein | 100 | 85.3 (▼ 14.7) | 92 (▲ 6.7) | 97.3 (▲ 5.3) | 102.6 (▲ 5.3) |
| ▲ and ▼ indicate an increase or a decrease of the percentage value of the corresponding column compared to the percentage value of immediately preceding column in each line. | | | | | |

**[Table 8]**

| Change in blood count of albumin administration group (based on the count of before bleeding, %) | | | | | |
|---|---|---|---|---|---|
| Category (%) | Recombinant feline albumin administration group | | | | |
| | Before bleeding (Baseline) | Before administration (after bleeding) | After administration (Day 1) | After administration (Day 3) | After administration (Day 7) |
| Hematocrit | 100 | 81.4 (▼ 18.6) | 93.6 (▲ 12.2) | 98 (▲ 4.4) | 101.7 (▲ 3.7) |
| Globulin | 100 | 94.7 (▼ 5.3) | 101.7 (▲ 7) | 104.4 (▲ 2.6) | 105.3 (▲ 0.9) |
| Albumin | 100 | 87.9 (▼ 12.1) | 101.3 (▲ 13.4) | 107.3 (▲ 6) | 109.8 (▲ 2.4) |

| Category (%) | Recombinant feline albumin administration group | | | | |
|---|---|---|---|---|---|
| | Before bleeding (Baseline) | Before administration (after bleeding) | After administration (Day 1) | After administration (Day 3) | After administration (Day 7) |
| Total protein | 100 | 91.5 (▼ 8.5) | 100.5 (▲ 9) | 104.3 (▲ 3.7) | 105.7 (▲ 1.4) |
| ▲ and ▼ indicate an increase or a decrease of the percentage value of the corresponding column compared to the percentage value of immediately preceding column in each line. | | | | | |

It was found that the large difference between Control and the recombinant feline albumin administration group is shown on Day 1 after injecting the test material, and, at that time, the increase rate of blood albumin is about 10.1% higher in the albumin administration group compared to Control, and it is maintained up to Day 7. Moreover, similar to the above test using dogs, no side effects were observed from the present test. Based on the above results, it is considered that the administration of recombinant feline albumin helps to have a higher albumin value without any side effect.

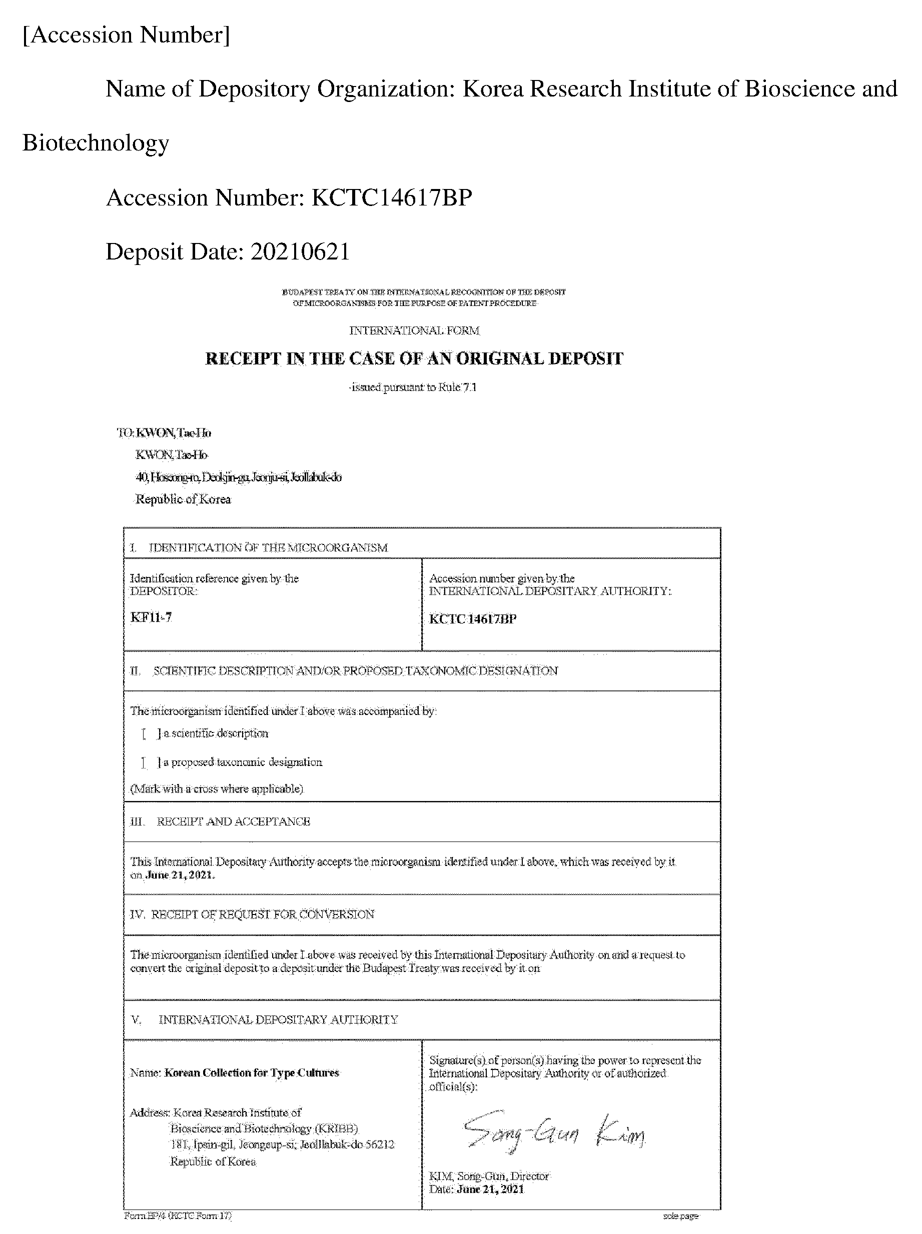
[Accession Number]

Name of Depository Organization: Korea Research Institute of Bioscience and

Biotechnology

Accession Number: KCTC14617BP

Deposit Date: 20210621

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KWON, Tae-Ho
KWON, Tae-Ho
40, Hosong-ro, Deokjin-gu, Jeonju-si, Jeollabuk-do
Republic of Korea

I. IDENTIFICATION OF THE MICROORGANISM
Identification reference given by the DEPOSITOR: | Accession number given by the INTERNATIONAL DEPOSITARY AUTHORITY:
KF11-7 | KCTC 14617BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it on June 21, 2021.
IV. RECEIPT OF REQUEST FOR CONVERSION
The microorganism identified under I above was received by this International Depositary Authority on and a request to convert the original deposit to a deposit under the Budapest Treaty was received by it on
V. INTERNATIONAL DEPOSITARY AUTHORITY
Name: Korean Collection for Type Cultures | Signature(s) of person(s) having the power to represent the International Depositary Authority or of authorized official(s):
Address: Korea Research Institute of Bioscience and Biotechnology (KRIBB) 181, Ipsin-gil, Jeongeup-si, Jeollabuk-do 56212 Republic of Korea | KIM, Song-Gun, Director Date: June 21, 2021

Form BP/4 (KCTC Form 17) sole page

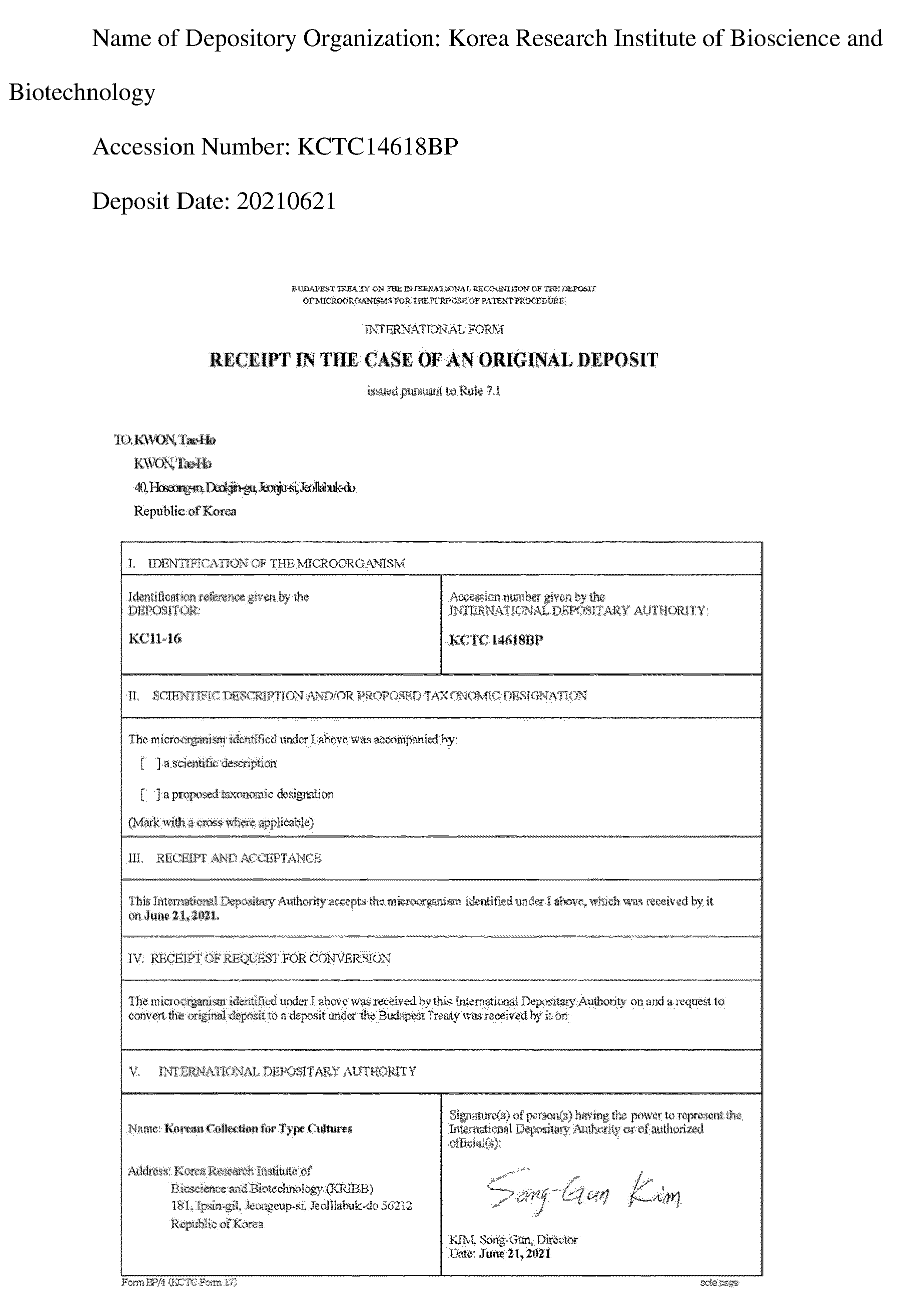
Name of Depository Organization: Korea Research Institute of Bioscience and
Biotechnology
Accession Number: KCTC14618BP
Deposit Date: 20210621

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT
issued pursuant to Rule 7.1

TO: KWON, Tae-Ho
KWON, Tae-Ho
40, Hosong-ro, Deokjin-gu, Jeonju-si, Jeollabuk-do
Republic of Korea

I. IDENTIFICATION OF THE MICROORGANISM
Identification reference given by the
DEPOSITOR:
KC11-16 | Accession number given by the
INTERNATIONAL DEPOSITARY AUTHORITY:
KCTC 14618BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on June 21, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: June 21, 2021
Form BP/4 (KCTC Form 17) sole page

## Claims

1. A recombinant nucleic acid for producing albumin at high efficiency in which a nucleic acid sequence encoding signal peptide (SP) is attached to 5'-terminus of albumin gene.

2. The recombinant nucleic acid according to Claim 1, wherein the albumin gene originates from a dog or a cat.

3. The recombinant nucleic acid according to Claim 1, wherein the albumin gene includes the nucleotide sequence of SEQ ID NO: 1 or SEQ ID NO: 2.

4. The recombinant nucleic acid according to Claim 1, wherein the signal peptide is derived from rice amylase 3A (RAmy3A) or soybean vegetative storage protein (VspA).

5. The recombinant nucleic acid according to Claim 4, wherein the signal peptide consists of the amino acid sequence of SEQ ID NO: 11 or SEQ ID NO: 12.

6. An expression vector operably linked with the recombinant nucleic acid of Claim 1.

7. The expression vector according to Claim 6, wherein it includes 5'-UTR, a nucleotide sequence encoding signal peptide, and a nucleotide sequence encoding mature peptide of canine albumin or feline albumin.

8. The expression vector according to Claim 6, wherein it includes any one nucleotide sequence selected from SEQ ID NO: 7 to SEQ ID NO: 10.

9. A host cell comprising the expression vector of Claim 6.

10. The host cell according to Claim 9, wherein it is a plant cell which produces recombinant canine albumin or recombinant feline albumin at high efficiency.

11. A method for producing albumin at high efficiency including culturing a host cell comprising the expression vector of Claim 6.

12. A veterinary composition for preventing or treating hypoalbuminemia comprising, as an effective component, the recombinant nucleic acid of Claim 1, the expression vector of Claim 6, the host cell of Claim 9, or recombinant albumin protein derived from them.

13. The veterinary composition for preventing or treating hypoalbuminemia according to Claim 12, wherein it is to be administered to a dog or a cat and has decreased side effects compared to those caused by administration of human albumin.

14. The veterinary composition for preventing or treating hypoalbuminemia according to Claim 13, wherein the side effects are edema, urticaria, or skin lesions.

15. An animal feed additive composition for preventing or ameliorating hypoalbuminemia comprising, as an effective component, the recombinant nucleic acid of Claim 1, the expression vector of Claim 6, the host cell of Claim 9, or recombinant albumin protein derived from them.
